# EUROPEAN PATENT APPLICATION

(11) **EP 1 988 162 A1**
(43) Date of publication of application: **05.11.2008**
(21) Application number: 07714862.5
(22) Date of filing: 23.02.2007
(51) Int. Cl.: C12N 15/00, A61K 9/127, A61K 39/395, A61P 35/00, C07K 16/30, C12N 1/15, C12N 1/19, C12N 1/21, C12N 5/10, C12N 15/09, G01N 33/577, C12P 21/08

(54) **MONOCLONAL ANTIBODY, GENE ENCODING THE ANTIBODY, HYBRIDOMA, PHARMACEUTICAL COMPOSITION, AND DIAGNOSTIC REAGENT**

(30) Priority: 23.02.2006 JP 2006046974
(71) Applicant: Mitsubishi Tanabe Pharma Corporation, Chuo-ku Osaka-shi Osaka 541-8505 (JP); Keio University, Tokyo 108-8345 (JP)
(72) Inventor: HOSOKAWA, Saiko, Chuo-ku Tokyo 103-8405 (JP); HIRAKAWA, Yoko, Chuo-ku Tokyo 103-8405 (JP); FUKUDA, Kazumasa, Shinjuku-ku Tokyo 160-8582 (JP); NAKAMURA, Rieko, Shinjuku-ku Tokyo 160-8582 (JP); SAIKAWA, Yoshiro, Shinjuku-ku Tokyo 160-8582 (JP); AOKI, Masahiko, Shinjuku-ku Tokyo 160-8582 (JP); KUMAI, Koichiro, Shinjuku-ku Tokyo 160-8582 (JP)
(74) Representative: Prüfer & Partner GbR European Patent Attorneys
(86) International application number: PCT/JP2007/053391
(87) International publication number: WO 2007/097418

(57) **Abstract**

Disclosed is a monoclonal antibody which has a heavy chain variable region containing amino acid sequences depicted in SEQ ID NOs:74, 76 and 78 and a light chain variable region containing amino acid sequences depicted in SEQ ID NOs:80, 82 and 84. The monoclonal antibody can be used as a cancer therapeutic agent which acts selectively on a cancer tissue of non-small lung cancer, pancreatic cancer, gastric cancer or the like.

SEQUENCE LISTING

<110>  Mitsubishi Pharma Corporation

<120>  MONOCLONAL ANTIBODY, GENE ENCODING THE ANTIBODY, HYBRIDOMA, PHARMACEUTICAL
COMPOSITION, AND DIAGNOSTIC REAGENT

<130>  MP5226-C6304

<150>  JP2006-046974
<151>  2006-02-23

<160>  106

<170>  PatentIn version 3.1

<210>  1
<211>  23
<212>  DNA
<213>  Homo sapiens

<220>
<223>  primer

<220>
<221>  CDS
<222>  (1)..(21)

<400>  1
cag gtg cag ctg gtg cag tct gg                                    23
Gln Val Gln Leu Val Gln Ser
1               5

<210>  2
<211>  7
<212>  PRT
<213>  Homo sapiens

<400>  2
Gln Val Gln Leu Val Gln Ser
1               5

<210>  3
<211>  23
<212>  DNA

<213>  Homo sapiens

<220>
<223>  primer

<220>
<221>  CDS
<222>  (1)..(21)

<400>  3
cag gtc aac tta agg gag tct gg                                          23
Gln Val Asn Leu Arg Glu Ser
1               5

<210>  4
<211>  7
<212>  PRT
<213>  Homo sapiens

<400>  4
Gln Val Asn Leu Arg Glu Ser
1               5

<210>  5
<211>  23
<212>  DNA
<213>  Homo sapiens

<220>
<223>  primer

<220>
<221>  CDS
<222>  (1)..(21)

<400>  5
gag gtg cag ctg gtg gag tct gg                                          23
Glu Val Gln Leu Val Glu Ser
1               5

<210>  6
<211>  7
<212>  PRT
<213>  Homo sapiens

<400> 6
Glu Val Gln Leu Val Glu Ser
1               5

<210> 7
<211> 23
<212> DNA
<213> Homo sapiens

<220>
<223> primer

<220>
<221> CDS
<222> (1)..(21)

<400> 7
cag gtg cag ctg cag gag tcg gg                                          23
Gln Val Gln Leu Gln Glu Ser
1               5

<210> 8
<211> 7
<212> PRT
<213> Homo sapiens

<400> 8
Gln Val Gln Leu Gln Glu Ser
1               5

<210> 9
<211> 23
<212> DNA
<213> Homo sapiens

<220>
<223> primer

<220>
<221> CDS
<222> (1)..(21)

<400> 9
gag gtg cag ctg ttg cag tct gc                                          23
Glu Val Gln Leu Leu Gln Ser

1               5

<210>  10
<211>  7
<212>  PRT
<213>  Homo sapiens

<400>  10
Glu Val Gln Leu Leu Gln Ser
1               5

<210>  11
<211>  23
<212>  DNA
<213>  Homo sapiens

<220>
<223>  primer

<220>
<221>  CDS
<222>  (1)..(21)

<400>  11
cag gta cag ctg cag cag tca gg                                          23
Gln Val Gln Leu Gln Gln Ser
1               5

<210>  12
<211>  7
<212>  PRT
<213>  Homo sapiens

<400>  12
Gln Val Gln Leu Gln Gln Ser
1               5

<210>  13
<211>  24
<212>  DNA
<213>  Homo sapiens

<220>
<223>  primer

<400>  13
tga gga gac ggt gac cag ggt gcc                                          24

<210>  14
<211>  8
<212>  PRT
<213>  Homo sapiens

<400>  14
Gly Thr Leu Val Thr Val Ser Ser
1               5

<210>  15
<211>  24
<212>  DNA
<213>  Homo sapiens

<220>
<223>  primer

<400>  15
tga aga gac ggt gac cat tgt ccc                                          24


<210>  16
<211>  8
<212>  PRT
<213>  Homo sapiens

<400>  16
Gly Thr Met Val Thr Val Ser Ser
1               5

<210>  17
<211>  24
<212>  DNA
<213>  Homo sapiens

<220>
<223>  primer

<400>  17
tga gga gac ggt gac cag ggt tcc                                          24

<210>  18

<211>  8
<212>  PRT
<213>  Homo sapiens

<400>  18
Gly Thr Leu Val Thr Val Ser Ser
1               5

<210>  19
<211>  24
<212>  DNA
<213>  Homo sapiens

<220>
<223>  primer

<400>  19
tga gga gac ggt gac cgt ggt ccc                                          24

<210>  20
<211>  8
<212>  PRT
<213>  Homo sapiens

<400>  20
Gly Thr Thr Val Thr Val Ser Ser
1               5

<210>  21
<211>  23
<212>  DNA
<213>  Homo sapiens

<220>
<223>  primer

<220>
<221>  CDS
<222>  (1)..(21)

<400>  21
gac atc cag atg acc cag tct cc                                           23
Asp Ile Gln Met Thr Gln Ser
1               5

<210>  22
<211>  7
<212>  PRT
<213>  Homo sapiens

<400>  22
Asp Ile Gln Met Thr Gln Ser
1               5

<210>  23
<211>  23
<212>  DNA
<213>  Homo sapiens

<220>
<223>  primer

<220>
<221>  CDS
<222>  (1)..(21)

<400>  23
gat gtt gtg atg act cag tct cc                                          23
Asp Val Val Met Thr Gln Ser
1               5

<210>  24
<211>  7
<212>  PRT
<213>  Homo sapiens

<400>  24
Asp Val Val Met Thr Gln Ser
1               5

<210>  25
<211>  23
<212>  DNA
<213>  Homo sapiens

<220>
<223>  primer

<220>
<221>  CDS

<222>  (1)..(21)

<400>  25
gaa att gtg ttg acg cag tct cc                                          23
Glu Ile Val Leu Thr Gln Ser
1               5

<210>  26
<211>  7
<212>  PRT
<213>  Homo sapiens

<400>  26
Glu Ile Val Leu Thr Gln Ser
1               5

<210>  27
<211>  23
<212>  DNA
<213>  Homo sapiens

<220>
<223>  primer

<220>
<221>  CDS
<222>  (1)..(21)

<400>  27
gac atc gtg atg acc cag tct cc                                          23
Asp Ile Val Met Thr Gln Ser
1               5

<210>  28
<211>  7
<212>  PRT
<213>  Homo sapiens

<400>  28
Asp Ile Val Met Thr Gln Ser
1               5

<210>  29
<211>  23
<212>  DNA

<213>  Homo sapiens

<220>
<223>  primer

<220>
<221>  CDS
<222>  (1)..(21)

<400>  29
gaa acg aca ctc acg cag tct cc                                          23
Glu Thr Thr Leu Thr Gln Ser
1               5

<210>  30
<211>  7
<212>  PRT
<213>  Homo sapiens

<400>  30
Glu Thr Thr Leu Thr Gln Ser
1               5

<210>  31
<211>  23
<212>  DNA
<213>  Homo sapiens

<220>
<223>  primer

<220>
<221>  CDS
<222>  (1)..(21)

<400>  31
gaa att gtg ctg act cag tct cc                                          23
Glu Ile Val Leu Thr Gln Ser
1               5

<210>  32
<211>  7
<212>  PRT
<213>  Homo sapiens

<400>  32
Glu Ile Val Leu Thr Gln Ser
1               5

<210>  33
<211>  24
<212>  DNA
<213>  Homo sapiens

<220>
<223>  primer

<400>  33
acg ttt gat ttc cac ctt ggt ccc                                          24

<210>  34
<211>  8
<212>  PRT
<213>  Homo sapiens

<400>  34
Gly Thr Lys Val Glu Ile Lys Arg
1               5

<210>  35
<211>  24
<212>  DNA
<213>  Homo sapiens

<220>
<223>  primer

<400>  35
acg ttt gat ctc cag ctt ggt ccc                                          24

<210>  36
<211>  8
<212>  PRT
<213>  Homo sapiens

<400>  36
Gly Thr Lys Leu Glu Ile Lys Arg
1               5

<210> 37
<211> 24
<212> DNA
<213> Homo sapiens

<220>
<223> primer

<400> 37
acg ttt gat atc cac ttt ggt ccc 24

<210> 38
<211> 8
<212> PRT
<213> Homo sapiens

<400> 38
Gly Thr Lys Val Asp Ile Lys Arg
1               5

<210> 39
<211> 24
<212> DNA
<213> Homo sapiens

<220>
<223> primer

<400> 39
acg ttt gat ctc cac ctt ggt ccc 24

<210> 40
<211> 8
<212> PRT
<213> Homo sapiens

<400> 40
Gly Thr Lys Val Glu Ile Lys Arg
1               5

<210> 41
<211> 24
<212> DNA
<213> Homo sapiens

<220>
<223>  primer

<400>  41
acg ttt aat ctc cag tcg tgt ccc                                          24

<210>  42
<211>  8
<212>  PRT
<213>  Homo sapiens

<400>  42
Gly Thr Arg Leu Glu Ile Lys Arg
1               5

<210>  43
<211>  23
<212>  DNA
<213>  Homo sapiens

<220>
<223>  primer

<220>
<221>  CDS
<222>  (1)..(21)

<400>  43
cag tct gtg ttg acg cag ccg cc                                           23
Gln Ser Val Leu Thr Gln Pro
1               5

<210>  44
<211>  7
<212>  PRT
<213>  Homo sapiens

<400>  44
Gln Ser Val Leu Thr Gln Pro
1               5

<210>  45
<211>  23
<212>  DNA

<213>  Homo sapiens

<220>
<223>  primer

<220>
<221>  CDS
<222>  (1)..(21)

<400>  45
cag tct gcc ctg act cag cct gc                                          23
Gln Ser Ala Leu Thr Gln Pro
1               5

<210>  46
<211>  7
<212>  PRT
<213>  Homo sapiens

<400>  46
Gln Ser Ala Leu Thr Gln Pro
1               5

<210>  47
<211>  23
<212>  DNA
<213>  Homo sapiens

<220>
<223>  primer

<220>
<221>  CDS
<222>  (1)..(21)

<400>  47
tcc tat gtg ctg act cag cca cc                                          23
Ser Tyr Val Leu Thr Gln Pro
1               5

<210>  48
<211>  7
<212>  PRT
<213>  Homo sapiens

<400>  48
Ser Tyr Val Leu Thr Gln Pro
1               5

<210>  49
<211>  23
<212>  DNA
<213>  Homo sapiens

<220>
<223>  primer

<220>
<221>  CDS
<222>  (1)..(21)

<400>  49
tct tct gag ctg act cag gac cc                                          23
Ser Ser Glu Leu Thr Gln Asp
1               5

<210>  50
<211>  7
<212>  PRT
<213>  Homo sapiens

<400>  50
Ser Ser Glu Leu Thr Gln Asp
1               5

<210>  51
<211>  23
<212>  DNA
<213>  Homo sapiens

<220>
<223>  primer

<220>
<221>  CDS
<222>  (1)..(21)

<400>  51
cac gtt ata ctg act caa ccg cc                                          23
His Val Ile Leu Thr Gln Pro

1                   5

<210>  52
<211>  7
<212>  PRT
<213>  Homo sapiens

<400>  52
His Val Ile Leu Thr Gln Pro
1                   5

<210>  53
<211>  23
<212>  DNA
<213>  Homo sapiens

<220>
<223>  primer

<220>
<221>  CDS
<222>  (1)..(21)

<400>  53
cag gct gtg ctc act cag ccg tc                                          23
Gln Ala Val Leu Thr Gln Pro
1                   5

<210>  54
<211>  7
<212>  PRT
<213>  Homo sapiens

<400>  54
Gln Ala Val Leu Thr Gln Pro
1                   5

<210>  55
<211>  23
<212>  DNA
<213>  Homo sapiens

<220>
<223>  primer

<220>
<221>  CDS
<222>  (1)..(21)

<400>  55
aat ttt atg ctg act cag ccc ca                                          23
Asn Phe Met Leu Thr Gln Pro
1               5

<210>  56
<211>  7
<212>  PRT
<213>  Homo sapiens

<400>  56
Asn Phe Met Leu Thr Gln Pro
1               5

<210>  57
<211>  24
<212>  DNA
<213>  Homo sapiens

<220>
<223>  Homo sapiens

<400>  57
acc tag gac ggt gac ctt ggt ccc                                         24

<210>  58
<211>  8
<212>  PRT
<213>  Homo sapiens

<400>  58
Gly Thr Lys Val Thr Val Leu Gly
1               5

<210>  59
<211>  24
<212>  DNA
<213>  Homo sapiens

<220>
<223>  primer

<400> 59
acc tag gac ggt cag ctt ggt ccc 24

<210> 60
<211> 8
<212> PRT
<213> Homo sapiens

<400> 60
Gly Thr Lys Leu Thr Val Leu Gly
1               5

<210> 61
<211> 24
<212> DNA
<213> Homo sapiens

<220>
<223> primer

<400> 61
acc taa aac ggt gag ctg ggt ccc 24

<210> 62
<211> 8
<212> PRT
<213> Homo sapiens

<400> 62
Gly Thr Gln Leu Thr Val Leu Gly
1               5

<210> 63
<211> 375
<212> DNA
<213> Homo sapiens

<220>
<221> CDS
<222> (1)..(375)

<400> 63
gag gtg cag ctg gtg gag tct ggg gga ggc ctg gtc aag cct ggg ggg 48
Glu Val Gln Leu Val Glu Ser Gly Gly Gly Leu Val Lys Pro Gly Gly

1               5                   10                  15
tcc ctg aga ctc tcc tgt gtt gcc tct gga ttc aca ttc aga gtc tat         96
Ser Leu Arg Leu Ser Cys Val Ala Ser Gly Phe Thr Phe Arg Val Tyr
            20                  25                  30
acc ata aac tgg gtc cgt cag gct cca ggg aag ggg ctg gag tgg gtc         144
Thr Ile Asn Trp Val Arg Gln Ala Pro Gly Lys Gly Leu Glu Trp Val
        35                  40                  45
tca tcc att agt gga agt ggc gct gtg ata tat tac gct gac tca gtg         192
Ser Ser Ile Ser Gly Ser Gly Ala Val Ile Tyr Tyr Ala Asp Ser Val
    50                  55                  60
aag ggc cgc ttc acc atc tcc aga gac aac tcc aag aac tca gtg tct         240
Lys Gly Arg Phe Thr Ile Ser Arg Asp Asn Ser Lys Asn Ser Val Ser
65                  70                  75                  80
ctg caa atg aac agc ctg aga gcc gaa gac acg gct gtg tat ttt tgt         288
Leu Gln Met Asn Ser Leu Arg Ala Glu Asp Thr Ala Val Tyr Phe Cys
                85                  90                  95
gcg cgg gat gga gag atg gcc aca act ccc cgg aac gac tac gct ttg         336
Ala Arg Asp Gly Glu Met Ala Thr Thr Pro Arg Asn Asp Tyr Ala Leu
            100                 105                 110
gac gtc tgg ggc caa ggg acc acg gtc acc gtc tcc tca                     375
Asp Val Trp Gly Gln Gly Thr Thr Val Thr Val Ser Ser
        115                 120                 125

<210>  64
<211>  125
<212>  PRT
<213>  Homo sapiens

<400>  64
Glu Val Gln Leu Val Glu Ser Gly Gly Gly Leu Val Lys Pro Gly Gly
1               5                   10                  15
Ser Leu Arg Leu Ser Cys Val Ala Ser Gly Phe Thr Phe Arg Val Tyr
            20                  25                  30
Thr Ile Asn Trp Val Arg Gln Ala Pro Gly Lys Gly Leu Glu Trp Val
        35                  40                  45
Ser Ser Ile Ser Gly Ser Gly Ala Val Ile Tyr Tyr Ala Asp Ser Val
    50                  55                  60
Lys Gly Arg Phe Thr Ile Ser Arg Asp Asn Ser Lys Asn Ser Val Ser
65                  70                  75                  80
Leu Gln Met Asn Ser Leu Arg Ala Glu Asp Thr Ala Val Tyr Phe Cys
                85                  90                  95
Ala Arg Asp Gly Glu Met Ala Thr Thr Pro Arg Asn Asp Tyr Ala Leu
            100                 105                 110
Asp Val Trp Gly Gln Gly Thr Thr Val Thr Val Ser Ser

115                    120                    125

<210>  65
<211>  375
<212>  DNA
<213>  Homo sapiens

<220>
<221>  CDS
<222>  (1)..(375)

<400>  65
gag gtg cag ctg gtg gag tct ggg gga ggc ctg gtc aag cct ggg ggg        48
Glu Val Gln Leu Val Glu Ser Gly Gly Gly Leu Val Lys Pro Gly Gly
1               5                   10                  15
tcc ctg aga ctc tcc tgt gtt gcc tct gga ttc aca ttc aga gtc tat        96
Ser Leu Arg Leu Ser Cys Val Ala Ser Gly Phe Thr Phe Arg Val Tyr
            20                  25                  30
acc ata aac tgg gtc cgt cag gct cca ggg aag ggg ctg gag tgg gtc       144
Thr Ile Asn Trp Val Arg Gln Ala Pro Gly Lys Gly Leu Glu Trp Val
        35                  40                  45
tca tcc act agt gga agt ggc gct gtg ata tat tac gct gac tca gtg       192
Ser Ser Thr Ser Gly Ser Gly Ala Val Ile Tyr Tyr Ala Asp Ser Val
    50                  55                  60
aag ggc cgc ttc acc atc tcc aga gac aac tcc aag aac tca gtg tct       240
Lys Gly Arg Phe Thr Ile Ser Arg Asp Asn Ser Lys Asn Ser Val Ser
65                  70                  75                  80
ctg caa atg aac agc ctg aga gcc gaa gac acg gct gtg tat ttt tgt       288
Leu Gln Met Asn Ser Leu Arg Ala Glu Asp Thr Ala Val Tyr Phe Cys
                85                  90                  95
gcg cgg gat gga gag atg gcc aca act ccc cgg aac gac tac gct ttg       336
Ala Arg Asp Gly Glu Met Ala Thr Thr Pro Arg Asn Asp Tyr Ala Leu
            100                 105                 110
gac gtc tgg ggc caa ggg acc acg gtc acc gtc tcc tca                   375
Asp Val Trp Gly Gln Gly Thr Thr Val Thr Val Ser Ser
        115                 120                 125

<210>  66
<211>  125
<212>  PRT
<213>  Homo sapiens

<400>  66

Glu Val Gln Leu Val Glu Ser Gly Gly Gly Leu Val Lys Pro Gly Gly
1               5                   10                  15
Ser Leu Arg Leu Ser Cys Val Ala Ser Gly Phe Thr Phe Arg Val Tyr
            20                  25                  30
Thr Ile Asn Trp Val Arg Gln Ala Pro Gly Lys Gly Leu Glu Trp Val
        35                  40                  45
Ser Ser Thr Ser Gly Ser Gly Ala Val Ile Tyr Tyr Ala Asp Ser Val
    50                  55                  60
Lys Gly Arg Phe Thr Ile Ser Arg Asp Asn Ser Lys Asn Ser Val Ser
65                  70                  75                  80
Leu Gln Met Asn Ser Leu Arg Ala Glu Asp Thr Ala Val Tyr Phe Cys
                85                  90                  95
Ala Arg Asp Gly Glu Met Ala Thr Thr Pro Arg Asn Asp Tyr Ala Leu
            100                 105                 110
Asp Val Trp Gly Gln Gly Thr Thr Val Thr Val Ser Ser
        115                 120                 125

<210>  67
<211>  375
<212>  DNA
<213>  Homo sapiens

<220>
<221>  CDS
<222>  (1)..(375)

<400>  67
gag gtg cag ctg gtg cag tct ggg gga ggc ctg gtc aag cct ggg ggg      48
Glu Val Gln Leu Val Gln Ser Gly Gly Gly Leu Val Lys Pro Gly Gly
1               5                   10                  15
tcc ctg aga ctc tcc tgt gtt gcc tct gga ttc aca ttc aga gtc tat      96
Ser Leu Arg Leu Ser Cys Val Ala Ser Gly Phe Thr Phe Arg Val Tyr
            20                  25                  30
acc ata aac tgg gtc cgt cag gct cca ggg aag ggg ctg gag tgg gtc      144
Thr Ile Asn Trp Val Arg Gln Ala Pro Gly Lys Gly Leu Glu Trp Val
        35                  40                  45
tca tcc att agt gga agt ggc gct gtg ata tat tac gct gac tca gtg      192
Ser Ser Ile Ser Gly Ser Gly Ala Val Ile Tyr Tyr Ala Asp Ser Val
    50                  55                  60
aat ggc cgc ttc acc atc tcc aga gac aac tcc aag aac tca gtg tct      240
Asn Gly Arg Phe Thr Ile Ser Arg Asp Asn Ser Lys Asn Ser Val Ser
65                  70                  75                  80
ctg caa atg aac agc ctg aga gcc gaa gac acg gct gtg tat ttt tgt      288
Leu Gln Met Asn Ser Leu Arg Ala Glu Asp Thr Ala Val Tyr Phe Cys

85                    90                    95
gcg cgg gat gga gag atg gcc aca act ccc cgg aac gac tac gct ttg        336
Ala Arg Asp Gly Glu Met Ala Thr Thr Pro Arg Asn Asp Tyr Ala Leu
            100                   105                   110
gac gtc tgg ggc caa ggg acc acg gtc acc gtc tcc tca                    375
Asp Val Trp Gly Gln Gly Thr Thr Val Thr Val Ser Ser
        115                   120                   125

<210>  68
<211>  125
<212>  PRT
<213>  Homo sapiens

<400>  68
Glu Val Gln Leu Val Gln Ser Gly Gly Gly Leu Val Lys Pro Gly Gly
1               5                   10                  15
Ser Leu Arg Leu Ser Cys Val Ala Ser Gly Phe Thr Phe Arg Val Tyr
            20                  25                  30
Thr Ile Asn Trp Val Arg Gln Ala Pro Gly Lys Gly Leu Glu Trp Val
        35                  40                  45
Ser Ser Ile Ser Gly Ser Gly Ala Val Ile Tyr Tyr Ala Asp Ser Val
    50                  55                  60
Asn Gly Arg Phe Thr Ile Ser Arg Asp Asn Ser Lys Asn Ser Val Ser
65                  70                  75                  80
Leu Gln Met Asn Ser Leu Arg Ala Glu Asp Thr Ala Val Tyr Phe Cys
                85                  90                  95
Ala Arg Asp Gly Glu Met Ala Thr Thr Pro Arg Asn Asp Tyr Ala Leu
            100                 105                 110
Asp Val Trp Gly Gln Gly Thr Thr Val Thr Val Ser Ser
        115                 120                 125

<210>  69
<211>  336
<212>  DNA
<213>  Homo sapiens

<220>
<221>  CDS
<222>  (1)..(336)

<400>  69
cag tct gtg ttg acg cag ccg ccc tca gtg tct ggg gcc cca ggg cag        48
Gln Ser Val Leu Thr Gln Pro Pro Ser Val Ser Gly Ala Pro Gly Gln

1                5                   10                  15
agg gtc acc atc tcc tgc act ggg agc agc tcc aac atc ggg gca ggt        96
Arg Val Thr Ile Ser Cys Thr Gly Ser Ser Ser Asn Ile Gly Ala Gly
            20                  25                  30
ttt gat gtt cac tgg tac cag cag ctt cca gga aca gcc ccc aaa ctc        144
Phe Asp Val His Trp Tyr Gln Gln Leu Pro Gly Thr Ala Pro Lys Leu
        35                  40                  45
ctc atc tat gtt aat acc aat cgg ccc tca ggg gtc cct gac cgg ttc        192
Leu Ile Tyr Val Asn Thr Asn Arg Pro Ser Gly Val Pro Asp Arg Phe
    50                  55                  60
tct ggc tcc aag tct ggc acc tca gcc tcc ctg gcc atc act ggg ctc        240
Ser Gly Ser Lys Ser Gly Thr Ser Ala Ser Leu Ala Ile Thr Gly Leu
65                  70                  75                  80
cag gct gag gat gag gct gat tat tac tgc cag tcc tat gac agc agc        288
Gln Ala Glu Asp Glu Ala Asp Tyr Tyr Cys Gln Ser Tyr Asp Ser Ser
                85                  90                  95
ctg agt ggt tgg gta ttc ggc ggg ggg acc cag ctc acc gtc cta ggt        336
Leu Ser Gly Trp Val Phe Gly Gly Gly Thr Gln Leu Thr Val Leu Gly
            100                 105                 110

<210>  70
<211>  112
<212>  PRT
<213>  Homo sapiens

<400>  70
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile Gly Ala Gly
            20                  25                  30
Phe Asp Val His Trp Tyr Gln Gln Leu Pro Gly Thr Ala Pro Lys Leu
        35                  40                  45
Leu Ile Tyr Val Asn Thr Asn Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Tyr Asp Ser Ser
                85                  90                  95
Leu Ser Gly Trp Val Phe Gly Gly Gly Thr Gln Leu Thr Val Leu Gly
            100                 105                 110

<210>  71
<211>  375
<212>  DNA

<213>  Homo sapiens

<220>
<221>  CDS
<222>  (1)..(375)

<220>
<221>  misc_feature
<222>  (16)..(16)
<223>  s=g or c

<400>  71
gag gtg cag ctg gtg sag tct ggg gga ggc ctg gtc aag cct ggg ggg      48
Glu Val Gln Leu Val Xaa Ser Gly Gly Gly Leu Val Lys Pro Gly Gly
1               5                   10                  15
tcc ctg aga ctc tcc tgt gtt gcc tct gga ttc aca ttc aga gtc tat      96
Ser Leu Arg Leu Ser Cys Val Ala Ser Gly Phe Thr Phe Arg Val Tyr
            20                  25                  30
acc ata aac tgg gtc cgt cag gct cca ggg aag ggg ctg gag tgg gtc      144
Thr Ile Asn Trp Val Arg Gln Ala Pro Gly Lys Gly Leu Glu Trp Val
        35                  40                  45
tca tcc ayt agt gga agt ggc gct gtg ata tat tac gct gac tca gtg      192
Ser Ser Xaa Ser Gly Ser Gly Ala Val Ile Tyr Tyr Ala Asp Ser Val
    50                  55                  60
aak ggc cgc ttc acc atc tcc aga gac aac tcc aag aac tca gtg tct      240
Xaa Gly Arg Phe Thr Ile Ser Arg Asp Asn Ser Lys Asn Ser Val Ser
65                  70                  75                  80
ctg caa atg aac agc ctg aga gcc gaa gac acg gct gtg tat ttt tgt      288
Leu Gln Met Asn Ser Leu Arg Ala Glu Asp Thr Ala Val Tyr Phe Cys
                85                  90                  95
gcg cgg gat gga gag atg gcc aca act ccc cgg aac gac tac gct ttg      336
Ala Arg Asp Gly Glu Met Ala Thr Thr Pro Arg Asn Asp Tyr Ala Leu
            100                 105                 110
gac gtc tgg ggc caa ggg acc acg gtc acc gtc tcc tca                  375
Asp Val Trp Gly Gln Gly Thr Thr Val Thr Val Ser Ser
        115                 120                 125

<210>  72
<211>  125
<212>  PRT
<213>  Homo sapiens

<220>

<221>  misc_feature
<222>  (6)..(6)
<223>  The 'Xaa' at location 6 stands for Glu, or Gln.

<220>
<221>  misc_feature
<222>  (51)..(51)
<223>  The 'Xaa' at location 51 stands for Thr, or Ile.

<220>
<221>  misc_feature
<222>  (65)..(65)
<223>  The 'Xaa' at location 65 stands for Lys, or Asn.

<400>  72
Glu Val Gln Leu Val Xaa Ser Gly Gly Gly Leu Val Lys Pro Gly Gly
1               5                   10                  15
Ser Leu Arg Leu Ser Cys Val Ala Ser Gly Phe Thr Phe Arg Val Tyr
            20                  25                  30
Thr Ile Asn Trp Val Arg Gln Ala Pro Gly Lys Gly Leu Glu Trp Val
        35                  40                  45
Ser Ser Xaa Ser Gly Ser Gly Ala Val Ile Tyr Tyr Ala Asp Ser Val
    50                  55                  60
Xaa Gly Arg Phe Thr Ile Ser Arg Asp Asn Ser Lys Asn Ser Val Ser
65                  70                  75                  80
Leu Gln Met Asn Ser Leu Arg Ala Glu Asp Thr Ala Val Tyr Phe Cys
                85                  90                  95
Ala Arg Asp Gly Glu Met Ala Thr Thr Pro Arg Asn Asp Tyr Ala Leu
            100                 105                 110
Asp Val Trp Gly Gln Gly Thr Thr Val Thr Val Ser Ser
        115                 120                 125

<210>  73
<211>  15
<212>  DNA
<213>  Homo sapiens

<220>
<221>  CDS
<222>  (1)..(15)

<400>  73
gtc tat acc ata aac                                                    15
Val Tyr Thr Ile Asn
1               5

<210> 74
<211> 5
<212> PRT
<213> Homo sapiens

<400> 74
Val Tyr Thr Ile Asn
1               5

<210> 75
<211> 51
<212> DNA
<213> Homo sapiens

<220>
<221> CDS
<222> (1)..(51)

<220>
<221> misc_feature
<222> (5)..(5)
<223> y=t or c

<400> 75
tcc ayt agt gga agt ggc gct gtg ata tat tac gct gac tca gtg aak 48
Ser Xaa Ser Gly Ser Gly Ala Val Ile Tyr Tyr Ala Asp Ser Val Xaa
1               5                   10                  15
ggc 51
Gly

<210> 76
<211> 17
<212> PRT
<213> Homo sapiens

<220>
<221> misc_feature
<222> (2)..(2)
<223> The 'Xaa' at location 2 stands for Thr, or Ile.

<220>
<221> misc_feature
<222> (16)..(16)
<223> The 'Xaa' at location 16 stands for Lys, or Asn.

<400> 76
Ser Xaa Ser Gly Ser Gly Ala Val Ile Tyr Tyr Ala Asp Ser Val Xaa
1               5                   10                  15
Gly

<210> 77
<211> 48
<212> DNA
<213> Homo sapiens

<220>
<221> CDS
<222> (1)..(48)

<400> 77
gat gga gag atg gcc aca act ccc cgg aac gac tac gct ttg gac gtc      48
Asp Gly Glu Met Ala Thr Thr Pro Arg Asn Asp Tyr Ala Leu Asp Val
1               5                   10                  15

<210> 78
<211> 16
<212> PRT
<213> Homo sapiens

<400> 78
Asp Gly Glu Met Ala Thr Thr Pro Arg Asn Asp Tyr Ala Leu Asp Val
1               5                   10                  15

<210> 79
<211> 42
<212> DNA
<213> Homo sapiens

<220>
<221> CDS
<222> (1)..(42)

<400> 79
act ggg agc agc tcc aac atc ggg gca ggt ttt gat gtt cac              42

Thr Gly Ser Ser Ser Asn Ile Gly Ala Gly Phe Asp Val His
1               5                   10

<210>  80
<211>  14
<212>  PRT
<213>  Homo sapiens

<400>  80
Thr Gly Ser Ser Ser Asn Ile Gly Ala Gly Phe Asp Val His
1               5                   10

<210>  81
<211>  21
<212>  DNA
<213>  Homo sapiens

<220>
<221>  CDS
<222>  (1)..(21)

<400>  81
gtt aat acc aat cgg ccc tca                                          21
Val Asn Thr Asn Arg Pro Ser
1               5

<210>  82
<211>  7
<212>  PRT
<213>  Homo sapiens

<400>  82
Val Asn Thr Asn Arg Pro Ser
1               5

<210>  83
<211>  33
<212>  DNA
<213>  Homo sapiens

<220>
<221>  CDS
<222>  (1)..(33)

<400>  83

cag tcc tat gac agc agc ctg agt ggt tgg gta                                    33
Gln Ser Tyr Asp Ser Ser Leu Ser Gly Trp Val
1               5                   10

<210>  84
<211>  11
<212>  PRT
<213>  Homo sapiens

<400>  84
Gln Ser Tyr Asp Ser Ser Leu Ser Gly Trp Val
1               5                   10

<210>  85
<211>  328
<212>  DNA
<213>  Homo sapiens

<220>
<221>  CDS
<222>  (2)..(328)

<400>  85
g gga ggc ctg gtc aag cct ggg ggg tcc ctg aga ctc tcc tgt gtt gcc      49
  Gly Gly Leu Val Lys Pro Gly Gly Ser Leu Arg Leu Ser Cys Val Ala
  1               5                   10                  15
tct gga ttc aca ttc aga gtc tat acc ata aac tgg gtc cgt cag gct        97
Ser Gly Phe Thr Phe Arg Val Tyr Thr Ile Asn Trp Val Arg Gln Ala
            20                  25                  30
cca ggg aag ggg ctg gag tgg gtc tca tcc att agt gga agt ggc gct       145
Pro Gly Lys Gly Leu Glu Trp Val Ser Ser Ile Ser Gly Ser Gly Ala
        35                  40                  45
gtg ata tat tac gct gac tca gtg aag ggc cgc ttc acc atc tcc aga       193
Val Ile Tyr Tyr Ala Asp Ser Val Lys Gly Arg Phe Thr Ile Ser Arg
    50                  55                  60
gac aac tcc aag aac tca gtg tct ctg caa atg aac agc ctg aga gcc       241
Asp Asn Ser Lys Asn Ser Val Ser Leu Gln Met Asn Ser Leu Arg Ala
65                  70                  75                  80
gaa gac acg gct gtg tat ttt tgt gcg cgg gat gga gag atg gcc aca       289
Glu Asp Thr Ala Val Tyr Phe Cys Ala Arg Asp Gly Glu Met Ala Thr
                85                  90                  95
act ccc cgg aac gac tac gct ttg gac gtc tgg ggc caa                   328
Thr Pro Arg Asn Asp Tyr Ala Leu Asp Val Trp Gly Gln
            100                 105

<210> 86
<211> 109
<212> PRT
<213> Homo sapiens

<400> 86
Gly Gly Leu Val Lys Pro Gly Gly Ser Leu Arg Leu Ser Cys Val Ala
1               5                   10                  15
Ser Gly Phe Thr Phe Arg Val Tyr Thr Ile Asn Trp Val Arg Gln Ala
            20                  25                  30
Pro Gly Lys Gly Leu Glu Trp Val Ser Ser Ile Ser Gly Ser Gly Ala
        35                  40                  45
Val Ile Tyr Tyr Ala Asp Ser Val Lys Gly Arg Phe Thr Ile Ser Arg
    50                  55                  60
Asp Asn Ser Lys Asn Ser Val Ser Leu Gln Met Asn Ser Leu Arg Ala
65                  70                  75                  80
Glu Asp Thr Ala Val Tyr Phe Cys Ala Arg Asp Gly Glu Met Ala Thr
                85                  90                  95
Thr Pro Arg Asn Asp Tyr Ala Leu Asp Val Trp Gly Gln
            100                 105

<210> 87
<211> 289
<212> DNA
<213> Homo sapiens

<220>
<221> CDS
<222> (2)..(289)

<400> 87
c tca gtg tct ggg gcc cca ggg cag agg gtc acc atc tcc tgc act ggg      49
  Ser Val Ser Gly Ala Pro Gly Gln Arg Val Thr Ile Ser Cys Thr Gly
  1               5                   10                  15
agc agc tcc aac atc ggg gca ggt ttt gat gtt cac tgg tac cag cag      97
Ser Ser Ser Asn Ile Gly Ala Gly Phe Asp Val His Trp Tyr Gln Gln
            20                  25                  30
ctt cca gga aca gcc ccc aaa ctc ctc atc tat gtt aat acc aat cgg      145
Leu Pro Gly Thr Ala Pro Lys Leu Leu Ile Tyr Val Asn Thr Asn Arg
        35                  40                  45
ccc tca ggg gtc cct gac cgg ttc tct ggc tcc aag tct ggc acc tca      193
Pro Ser Gly Val Pro Asp Arg Phe Ser Gly Ser Lys Ser Gly Thr Ser
    50                  55                  60
gcc tcc ctg gcc atc act ggg ctc cag gct gag gat gag gct gat tat      241
Ala Ser Leu Ala Ile Thr Gly Leu Gln Ala Glu Asp Glu Ala Asp Tyr

65                  70                  75                  80
tac tgc cag tcc tat gac agc agc ctg agt ggt tgg gta ttc ggc ggg        289
Tyr Cys Gln Ser Tyr Asp Ser Ser Leu Ser Gly Trp Val Phe Gly Gly
                85                  90                  95

<210>  88
<211>  96
<212>  PRT
<213>  Homo sapiens

<400>  88
Ser Val Ser Gly Ala Pro Gly Gln Arg Val Thr Ile Ser Cys Thr Gly
1               5                   10                  15
Ser Ser Ser Asn Ile Gly Ala Gly Phe Asp Val His Trp Tyr Gln Gln
            20                  25                  30
Leu Pro Gly Thr Ala Pro Lys Leu Leu Ile Tyr Val Asn Thr Asn Arg
        35                  40                  45
Pro Ser Gly Val Pro Asp Arg Phe Ser Gly Ser Lys Ser Gly Thr Ser
    50                  55                  60
Ala Ser Leu Ala Ile Thr Gly Leu Gln Ala Glu Asp Glu Ala Asp Tyr
65                  70                  75                  80
Tyr Cys Gln Ser Tyr Asp Ser Ser Leu Ser Gly Trp Val Phe Gly Gly
                85                  90                  95

<210>  89
<211>  33
<212>  DNA
<213>  Artificial sequence

<220>
<223>  primer

<400>  89
ccccttaagc gaggtgcagc tggtggagtc tgg                                   33

<210>  90
<211>  33
<212>  DNA
<213>  Artificial sequence

<220>
<223>  primer

<400>  90
cccgctagct gaggagacgg tgaccgtggt ccc                                   33

<210> 91
<211> 47
<212> DNA
<213> Artificial sequence

<220>
<223> primer

<400> 91
cccccctgta caggctctga ggcccagtct gtgttgacgc agccgcc 47

<210> 92
<211> 47
<212> DNA
<213> Artificial sequence

<220>
<223> primer

<400> 92
cccccctgta caggctctga ggcccagtct gccctgactc agcctgc 47

<210> 93
<211> 47
<212> DNA
<213> Artificial sequence

<220>
<223> primer

<400> 93
cccccctgta caggctctga ggcctcctat gtgctgactc agccacc 47

<210> 94
<211> 47
<212> DNA
<213> Artificial sequence

<220>
<223> primer

<400> 94
cccccctgta caggctctga ggcctcttct gagctgactc aggaccc 47

<210> 95
<211> 47
<212> DNA
<213> Artificial sequence

<220>
<223> primer

<400> 95
cccccctgta caggctctga ggcccacgtt atactgactc aaccgcc 47

<210> 96
<211> 47
<212> DNA
<213> Artificial sequence

<220>
<223> primer

<400> 96
cccccctgta caggctctga ggcccaggct gtgctcactc agccgtc 47

<210> 97
<211> 47
<212> DNA
<213> Artificial sequence

<220>
<223> primer

<400> 97
cccccctgta caggctctga ggccaatttt atgctgactc agcccca 47

<210> 98
<211> 29
<212> DNA
<213> Artificial sequence

<220>
<223> primer

<400> 98
gggggggccta ggacggtgac cttggtccc 29

<210> 99

<211>  29
<212>  DNA
<213>  Artificial sequence

<220>
<223>  primer

<400>  99
gggggaccta ggacggtcag cttggtccc                                          29

<210>  100
<211>  29
<212>  DNA
<213>  Artificial sequence

<220>
<223>  primer

<400>  100
ggggggccta ggacggtgag ctgggtccc                                          29

<210>  101
<211>  22
<212>  DNA
<213>  Artificial sequence

<220>
<223>  primer

<400>  101
cccactgctt actggcttat cg                                                 22

<210>  102
<211>  18
<212>  DNA
<213>  Artificial sequence

<220>
<223>  primer

<400>  102
ggtgctcttg gaggaggg                                                      18

<210> 103
<211> 1425
<212> DNA
<213> Homo sapiens

<220>
<221> CDS
<222> (1)..(1422)

<220>
<221> misc_feature
<222> (73)..(73)
<223> s=g or c

<400> 103
atg aaa cac ctg tgg ttc ttc ctc ctg ctg gtg gca gct ccc aga tgg 48
Met Lys His Leu Trp Phe Phe Leu Leu Leu Val Ala Ala Pro Arg Trp
1               5                   10                  15
gtc tta agc gag gtg cag ctg gtg sag tct ggg gga ggc ctg gtc aag 96
Val Leu Ser Glu Val Gln Leu Val Xaa Ser Gly Gly Gly Leu Val Lys
            20                  25                  30
cct ggg ggg tcc ctg aga ctc tcc tgt gtt gcc tct gga ttc aca ttc 144
Pro Gly Gly Ser Leu Arg Leu Ser Cys Val Ala Ser Gly Phe Thr Phe
        35                  40                  45
aga gtc tat acc ata aac tgg gtc cgt cag gct cca ggg aag ggg ctg 192
Arg Val Tyr Thr Ile Asn Trp Val Arg Gln Ala Pro Gly Lys Gly Leu
    50                  55                  60
gag tgg gtc tca tcc ayt agt gga agt ggc gct gtg ata tat tac gct 240
Glu Trp Val Ser Ser Xaa Ser Gly Ser Gly Ala Val Ile Tyr Tyr Ala
65                  70                  75                  80
gac tca gtg aak ggc cgc ttc acc atc tcc aga gac aac tcc aag aac 288
Asp Ser Val Xaa Gly Arg Phe Thr Ile Ser Arg Asp Asn Ser Lys Asn
                85                  90                  95
tca gtg tct ctg caa atg aac agc ctg aga gcc gaa gac acg gct gtg 336
Ser Val Ser Leu Gln Met Asn Ser Leu Arg Ala Glu Asp Thr Ala Val
            100                 105                 110
tat ttt tgt gcg cgg gat gga gag atg gcc aca act ccc cgg aac gac 384
Tyr Phe Cys Ala Arg Asp Gly Glu Met Ala Thr Thr Pro Arg Asn Asp
        115                 120                 125
tac gct ttg gac gtc tgg ggc caa ggg acc acg gtc acc gtc tcc tca 432
Tyr Ala Leu Asp Val Trp Gly Gln Gly Thr Thr Val Thr Val Ser Ser
    130                 135                 140
gct agt acc aag ggc cca tcc gtc ttc ccc ctg gca ccc tcc tcc aag 480
Ala Ser Thr Lys Gly Pro Ser Val Phe Pro Leu Ala Pro Ser Ser Lys

145                 150                 155                 160
agc acc tct ggg ggc aca gcg gcc ctg ggc tgc ctg gtc aag gac tac      528
Ser Thr Ser Gly Gly Thr Ala Ala Leu Gly Cys Leu Val Lys Asp Tyr
                165                 170                 175
ttc cct gaa ccg gtg acg gtg tcg tgg aac tca ggc gcc ctg acc agc      576
Phe Pro Glu Pro Val Thr Val Ser Trp Asn Ser Gly Ala Leu Thr Ser
            180                 185                 190
ggc gtg cac acc ttc ccg gct gtc cta cag tcc tca gga ctc tac tcc      624
Gly Val His Thr Phe Pro Ala Val Leu Gln Ser Ser Gly Leu Tyr Ser
        195                 200                 205
ctc agc agc gtg gtg acc gtg ccc tcc agc agc ttg ggc acc cag acc      672
Leu Ser Ser Val Val Thr Val Pro Ser Ser Ser Leu Gly Thr Gln Thr
    210                 215                 220
tac atc tgc aac gtg aat cac aag ccc agc aac acc aag gtg gac aag      720
Tyr Ile Cys Asn Val Asn His Lys Pro Ser Asn Thr Lys Val Asp Lys
225                 230                 235                 240
aaa gtt gag ccc aaa tct tgt gac aaa act cac aca tgc cca ccg tgc      768
Lys Val Glu Pro Lys Ser Cys Asp Lys Thr His Thr Cys Pro Pro Cys
                245                 250                 255
cca gca cct gaa ctc ctg ggg gga ccg tca gtc ttc ctc ttc ccc cca      816
Pro Ala Pro Glu Leu Leu Gly Gly Pro Ser Val Phe Leu Phe Pro Pro
            260                 265                 270
aaa ccc aag gac acc ctc atg atc tcc cgg acc cct gag gtc aca tgc      864
Lys Pro Lys Asp Thr Leu Met Ile Ser Arg Thr Pro Glu Val Thr Cys
        275                 280                 285
gtg gtg gtg gac gtg agc cac gaa gac cct gag gtc aag ttc aac tgg      912
Val Val Val Asp Val Ser His Glu Asp Pro Glu Val Lys Phe Asn Trp
    290                 295                 300
tac gtg gac ggc gtg gag gtg cat aat gcc aag aca aag ccg cgg gag      960
Tyr Val Asp Gly Val Glu Val His Asn Ala Lys Thr Lys Pro Arg Glu
305                 310                 315                 320
gag cag tac aac agc acg tac cgt gtg gtc agc gtc ctc acc gtc ctg      1008
Glu Gln Tyr Asn Ser Thr Tyr Arg Val Val Ser Val Leu Thr Val Leu
                325                 330                 335
cac cag gac tgg ctg aat ggc aag gag tac aag tgc aag gtc tcc aac      1056
His Gln Asp Trp Leu Asn Gly Lys Glu Tyr Lys Cys Lys Val Ser Asn
            340                 345                 350
aaa gcc ctc cca gcc ccc atc gag aaa acc atc tcc aaa gcc aaa ggg      1104
Lys Ala Leu Pro Ala Pro Ile Glu Lys Thr Ile Ser Lys Ala Lys Gly
        355                 360                 365
cag ccc cga gaa cca cag gtg tac acc ctg ccc cca tcc cgg gat gag      1152
Gln Pro Arg Glu Pro Gln Val Tyr Thr Leu Pro Pro Ser Arg Asp Glu
    370                 375                 380
ctg acc aag aac cag gtc agc ctg acc tgc ctg gtc aaa ggc ttc tat      1200
Leu Thr Lys Asn Gln Val Ser Leu Thr Cys Leu Val Lys Gly Phe Tyr

385                 390                 395                 400
ccc agc gac atc gcc gtg gag tgg gag agc aat ggg cag ccg gag aac      1248
Pro Ser Asp Ile Ala Val Glu Trp Glu Ser Asn Gly Gln Pro Glu Asn
                405                 410                 415
aac tac aag acc acg cct ccc gtg ctg gac tcc gac ggc tcc ttc ttc      1296
Asn Tyr Lys Thr Thr Pro Pro Val Leu Asp Ser Asp Gly Ser Phe Phe
            420                 425                 430
ctc tac agc aag ctc acc gtg gac aag agc agg tgg cag cag ggg aac      1344
Leu Tyr Ser Lys Leu Thr Val Asp Lys Ser Arg Trp Gln Gln Gly Asn
        435                 440                 445
gtc ttc tca tgc tcc gtg atg cat gag gct ctg cac aac cac tac acg      1392
Val Phe Ser Cys Ser Val Met His Glu Ala Leu His Asn His Tyr Thr
    450                 455                 460
cag aag agc ctc tcc ctg tct ccg ggt aaa tga                          1425
Gln Lys Ser Leu Ser Leu Ser Pro Gly Lys
465                 470

<210>  104
<211>  474
<212>  PRT
<213>  Homo sapiens

<220>
<221>  misc_feature
<222>  (25)..(25)
<223>  The 'Xaa' at location 25 stands for Glu, or Gln.

<220>
<221>  misc_feature
<222>  (70)..(70)
<223>  The 'Xaa' at location 70 stands for Thr, or Ile.

<220>
<221>  misc_feature
<222>  (84)..(84)
<223>  The 'Xaa' at location 84 stands for Lys, or Asn.

<400>  104
Met Lys His Leu Trp Phe Phe Leu Leu Leu Val Ala Ala Pro Arg Trp
1               5                   10                  15
Val Leu Ser Glu Val Gln Leu Val Xaa Ser Gly Gly Gly Leu Val Lys
            20                  25                  30
Pro Gly Gly Ser Leu Arg Leu Ser Cys Val Ala Ser Gly Phe Thr Phe
        35                  40                  45

Arg Val Tyr Thr Ile Asn Trp Val Arg Gln Ala Pro Gly Lys Gly Leu
50                  55                  60
Glu Trp Val Ser Ser Xaa Ser Gly Ser Gly Ala Val Ile Tyr Tyr Ala
65                  70                  75                  80
Asp Ser Val Xaa Gly Arg Phe Thr Ile Ser Arg Asp Asn Ser Lys Asn
                85                  90                  95
Ser Val Ser Leu Gln Met Asn Ser Leu Arg Ala Glu Asp Thr Ala Val
            100                 105                 110
Tyr Phe Cys Ala Arg Asp Gly Glu Met Ala Thr Thr Pro Arg Asn Asp
        115                 120                 125
Tyr Ala Leu Asp Val Trp Gly Gln Gly Thr Thr Val Thr Val Ser Ser
    130                 135                 140
Ala Ser Thr Lys Gly Pro Ser Val Phe Pro Leu Ala Pro Ser Ser Lys
145                 150                 155                 160
Ser Thr Ser Gly Gly Thr Ala Ala Leu Gly Cys Leu Val Lys Asp Tyr
                165                 170                 175
Phe Pro Glu Pro Val Thr Val Ser Trp Asn Ser Gly Ala Leu Thr Ser
            180                 185                 190
Gly Val His Thr Phe Pro Ala Val Leu Gln Ser Ser Gly Leu Tyr Ser
        195                 200                 205
Leu Ser Ser Val Val Thr Val Pro Ser Ser Ser Leu Gly Thr Gln Thr
    210                 215                 220
Tyr Ile Cys Asn Val Asn His Lys Pro Ser Asn Thr Lys Val Asp Lys
225                 230                 235                 240
Lys Val Glu Pro Lys Ser Cys Asp Lys Thr His Thr Cys Pro Pro Cys
                245                 250                 255
Pro Ala Pro Glu Leu Leu Gly Gly Pro Ser Val Phe Leu Phe Pro Pro
            260                 265                 270
Lys Pro Lys Asp Thr Leu Met Ile Ser Arg Thr Pro Glu Val Thr Cys
        275                 280                 285
Val Val Val Asp Val Ser His Glu Asp Pro Glu Val Lys Phe Asn Trp
    290                 295                 300
Tyr Val Asp Gly Val Glu Val His Asn Ala Lys Thr Lys Pro Arg Glu
305                 310                 315                 320
Glu Gln Tyr Asn Ser Thr Tyr Arg Val Val Ser Val Leu Thr Val Leu
                325                 330                 335
His Gln Asp Trp Leu Asn Gly Lys Glu Tyr Lys Cys Lys Val Ser Asn
            340                 345                 350
Lys Ala Leu Pro Ala Pro Ile Glu Lys Thr Ile Ser Lys Ala Lys Gly
        355                 360                 365
Gln Pro Arg Glu Pro Gln Val Tyr Thr Leu Pro Pro Ser Arg Asp Glu
    370                 375                 380
Leu Thr Lys Asn Gln Val Ser Leu Thr Cys Leu Val Lys Gly Phe Tyr
385                 390                 395                 400
Pro Ser Asp Ile Ala Val Glu Trp Glu Ser Asn Gly Gln Pro Glu Asn

405                 410                 415
Asn Tyr Lys Thr Thr Pro Pro Val Leu Asp Ser Asp Gly Ser Phe Phe
            420                 425                 430
Leu Tyr Ser Lys Leu Thr Val Asp Lys Ser Arg Trp Gln Gln Gly Asn
        435                 440                 445
Val Phe Ser Cys Ser Val Met His Glu Ala Leu His Asn His Tyr Thr
    450                 455                 460
Gln Lys Ser Leu Ser Leu Ser Pro Gly Lys
465                 470

<210>  105
<211>  711
<212>  DNA
<213>  Homo sapiens

<220>
<221>  CDS
<222>  (1)..(708)

<400>  105
atg gcc tgg att cct cta ctt ctc ccc ctc ctc act ctc tgt aca ggc      48
Met Ala Trp Ile Pro Leu Leu Leu Pro Leu Leu Thr Leu Cys Thr Gly
1               5                   10                  15
tct gag gcc cag tct gtg ttg acg cag ccg ccc tca gtg tct ggg gcc      96
Ser Glu Ala Gln Ser Val Leu Thr Gln Pro Pro Ser Val Ser Gly Ala
            20                  25                  30
cca ggg cag agg gtc acc atc tcc tgc act ggg agc agc tcc aac atc      144
Pro Gly Gln Arg Val Thr Ile Ser Cys Thr Gly Ser Ser Ser Asn Ile
        35                  40                  45
ggg gca ggt ttt gat gtt cac tgg tac cag cag ctt cca gga aca gcc      192
Gly Ala Gly Phe Asp Val His Trp Tyr Gln Gln Leu Pro Gly Thr Ala
    50                  55                  60
ccc aaa ctc ctc atc tat gtt aat acc aat cgg ccc tca ggg gtc cct      240
Pro Lys Leu Leu Ile Tyr Val Asn Thr Asn Arg Pro Ser Gly Val Pro
65                  70                  75                  80
gac cgg ttc tct ggc tcc aag tct ggc acc tca gcc tcc ctg gcc atc      288
Asp Arg Phe Ser Gly Ser Lys Ser Gly Thr Ser Ala Ser Leu Ala Ile
                85                  90                  95
act ggg ctc cag gct gag gat gag gct gat tat tac tgc cag tcc tat      336
Thr Gly Leu Gln Ala Glu Asp Glu Ala Asp Tyr Tyr Cys Gln Ser Tyr
            100                 105                 110
gac agc agc ctg agt ggt tgg gta ttc ggc ggg ggg acc cag ctc acc      384
Asp Ser Ser Leu Ser Gly Trp Val Phe Gly Gly Gly Thr Gln Leu Thr
        115                 120                 125

gtc cta ggt cag ccc aag gct gcc ccc tcg gtc act ctg ttc ccg ccc     432
Val Leu Gly Gln Pro Lys Ala Ala Pro Ser Val Thr Leu Phe Pro Pro
    130                 135                 140
tcc tct gag gag ctt caa gcc aac aag gcc aca ctg gtg tgt ctc ata     480
Ser Ser Glu Glu Leu Gln Ala Asn Lys Ala Thr Leu Val Cys Leu Ile
145                 150                 155                 160
agt gac ttc tac ccg gga gcc gtg aca gtg gcc tgg aag gca gat agc     528
Ser Asp Phe Tyr Pro Gly Ala Val Thr Val Ala Trp Lys Ala Asp Ser
                165                 170                 175
agc ccc gtc aag gcg gga gtg gag acc acc aca ccc tcc aaa caa agc     576
Ser Pro Val Lys Ala Gly Val Glu Thr Thr Thr Pro Ser Lys Gln Ser
            180                 185                 190
aac aac aag tac gcg gcc agc agc tac ctg agc ctg acg cct gag cag     624
Asn Asn Lys Tyr Ala Ala Ser Ser Tyr Leu Ser Leu Thr Pro Glu Gln
        195                 200                 205
tgg aag tcc cac aga agc tac agc tgc cag gtc acg cat gaa ggg agc     672
Trp Lys Ser His Arg Ser Tyr Ser Cys Gln Val Thr His Glu Gly Ser
    210                 215                 220
acc gtg gag aag aca gtg gcc cct aca gaa tgt tca tag                 711
Thr Val Glu Lys Thr Val Ala Pro Thr Glu Cys Ser
225                 230                 235

<210>  106
<211>  236
<212>  PRT
<213>  Homo sapiens

<400>  106
Met Ala Trp Ile Pro Leu Leu Leu Pro Leu Leu Thr Leu Cys Thr Gly
1               5                   10                  15
Ser Glu Ala Gln Ser Val Leu Thr Gln Pro Pro Ser Val Ser Gly Ala
            20                  25                  30
Pro Gly Gln Arg Val Thr Ile Ser Cys Thr Gly Ser Ser Ser Asn Ile
        35                  40                  45
Gly Ala Gly Phe Asp Val His Trp Tyr Gln Gln Leu Pro Gly Thr Ala
    50                  55                  60
Pro Lys Leu Leu Ile Tyr Val Asn Thr Asn Arg Pro Ser Gly Val Pro
65                  70                  75                  80
Asp Arg Phe Ser Gly Ser Lys Ser Gly Thr Ser Ala Ser Leu Ala Ile
                85                  90                  95
Thr Gly Leu Gln Ala Glu Asp Glu Ala Asp Tyr Tyr Cys Gln Ser Tyr
            100                 105                 110
Asp Ser Ser Leu Ser Gly Trp Val Phe Gly Gly Gly Thr Gln Leu Thr
        115                 120                 125

Val Leu Gly Gln Pro Lys Ala Ala Pro Ser Val Thr Leu Phe Pro Pro
    130                 135                 140
Ser Ser Glu Glu Leu Gln Ala Asn Lys Ala Thr Leu Val Cys Leu Ile
145                 150                 155                 160
Ser Asp Phe Tyr Pro Gly Ala Val Thr Val Ala Trp Lys Ala Asp Ser
                165                 170                 175
Ser Pro Val Lys Ala Gly Val Glu Thr Thr Thr Pro Ser Lys Gln Ser
            180                 185                 190
Asn Asn Lys Tyr Ala Ala Ser Ser Tyr Leu Ser Leu Thr Pro Glu Gln
        195                 200                 205
Trp Lys Ser His Arg Ser Tyr Ser Cys Gln Val Thr His Glu Gly Ser
    210                 215                 220
Thr Val Glu Lys Thr Val Ala Pro Thr Glu Cys Ser
225                 230                 235

## Description

### TECHNICAL FIELD

The present invention relates to a novel monoclonal antibody useful for diagnosis and therapy of cancer, and a DNA encoding such a monoclonal antibody, a hybridoma producing such an antibody, and a pharmaceutical composition and a diagnostic reagent, each of which contains such an antibody.

### BACKGROUND ART

In the field of cancer therapy, targeting therapy against a specific type of cancer cell has been studied so far for the treatment of solid cancer on which no therapeutic agent shows sufficient effect. In such targeting therapy, a monoclonal antibody that specifically recognizes cancer cells is effective. However, the use of a mouse monoclonal antibody has some problems such as difficulty of repetitive administration because of side effects such as anaphylaxis caused by an immune response (Non-patent document 1).

For solving such problems, attempts have been conducted to obtain monoclonal antibodies with reduced side effects. A technology for producing a chimeric antibody in which constant region is replaced with that of human antibody by genetic engineering, a technology for producing a humanized antibody in which all regions except for hypervariable regions are replaced with those of human antibody, etc. are known. However, a complete human monoclonal antibody has been desired from the viewpoint of reducing side effects. As a method of obtaining a complete human monoclonal antibody, there is a hybridoma method using a lymphocyte derived from human (Non-patent document 2). Although there is a few report about a human monoclonal antibody that reacts cancer cells (Patent document 1), preparation of human monoclonal antibodies which adequately react with cancer cells has been still very difficult because of the reasons that it is very difficult to conduct passive immunity for the purpose of obtaining human B cells which produce a desired antibody, and that any efficient methodology which allows infinite reproduction of antibody-producing cells has not been established yet.

Even under such circumstances, some monoclonal antibodies which exhibit a killing-effect or anti-proliferative effect on specific cancer cells by itself or in combination with anti-cancer drugs have been developed using a humanized antibody technology or the like. In recent years, application of an anti-Her2-humanized antibody (HERCEPTIN) to breast cancer (Non-patent document 3), clinical trials using an anti-EGF receptor antibody (Non-patent document 4), or an anti-VEGF (vascular endothelial growth factor) antibody (Non-patent document 5), and the like have been reported. However, any antibody, which can be used for targeting therapy for cancers including a non-small cell lung cancer from which many patients are suffering or refractory cancers such as pancreatic cancer, has not yet been developed. For treating such types of cancer, the acquisition of a monoclonal antibody having high specificity to cancer tissue with reduced side effects has been desired.
Patent document 2 discloses a monoclonal antibody having high specificity to cancer tissues as such an antibody, more antibodies has been desired to be obtained.

[Patent document 1] JP3236667
[Patent document 2] JP2005-040126
[Non-patent document 1] Proc.Nat1.Acad.Sci.U.S.A. vol.86, p4220, 1989
[Non-patent document 2] Cancer Res. vol.45, p263, 1985
[Non-patent document 3] Oncology vol. 63 Suppl 1, pp 25-32, 2002
[Non-patent document 4] Semin Oncol. vol. 29, No. 5 Suppl 14, pp 18-30, 2002
[Non-patent document 5] Semin Oncol. vol. 29, No. 6 Suppl 16, pp 10-14, 2002
[Non-patent document 6] Mol Cell Biol. 1987, vol. 7, No. 11, p3908-15

### DISCLOSURE OF THE INVENTION

An object of the present invention is to provide a monoclonal antibody useful for diagnosis and treatment of cancer, particularly of non-small cell lung cancer, pancreatic cancer, and gastric cancer with reduced side effects.

The inventors of the present invention have made extensive studies to provide a monoclonal antibody that can be used in targeting therapy on cancer tissues. As a result, they prepared hybridoma cells that produce a novel human monoclonal antibody capable of specifically recognizing cancer cells such as HLC-1, PANC-1, HT29 and MKN45, and found that anti-cancer drug useful in targeting therapy can be obtained by using such an antibody. Accordingly, they completed the present invention.

That is, the present invention provides the followings.
(1) A monoclonal antibody, wherein variable region of heavy chain of said monoclonal antibody comprises amino acid sequences of SEQ ID NOS: 74, 76 and 78.
(2) The monoclonal antibody according to (1), wherein the variable region of the heavy chain comprises an amino acid sequence of SEQ ID NO: 72.
(3) The monoclonal antibody according to (1), wherein the variable region of the heavy chain comprises an amino acid sequence of SEQ ID NO: 104.
(4) A monoclonal antibody, wherein variable region of the light chain of said monoclonal antibody comprises amino acid sequences of SEQ ID NOS: 80, 82 and 84.
(5) The monoclonal antibody according to (4), wherein the variable region of the light chain comprises an amino acid sequence of SEQ ID NO: 70.
(6) The monoclonal antibody according to (4), wherein the variable region of the light chain comprises an amino acid sequence of SEQ ID NO: 106.
(7) A monoclonal antibody, wherein variable region of the heavy chain of said monoclonal antibody comprises amino acid sequences of SEQ ID NOS: 74, 76 and 78, and variable region of the light chain of said monoclonal antibody comprises amino acid sequences of SEQ ID NOS: 80, 82 and 84.
(8) The monoclonal antibody according to (7), wherein the variable region of the heavy chain contains an amino acid sequence of SEQ ID NO: 72, and the variable region of the light chain comprises an amino acid sequence of SEQ ID NO: 70.
(9) The monoclonal antibody according to (7), wherein the variable region of the heavy chain comprises an amino acid sequence of SEQ ID NO: 104, and the variable region of the light chain comprises an amino acid sequence of SEQ ID NO: 106.
(10) The monoclonal antibody according to any one of (1) to (9), wherein said monoclonal antibody is a human antibody.
(11) A DNA which encodes the monoclonal antibody according to any one of (1) to (10).
(12) The DNA according to (11), wherein a region that encodes variable region of the heavy chain comprises nucleotide sequences of SEQ ID NOS: 73, 75 and 77, and a region that encodes variable region of the light chain comprises nucleotide sequences of SEQ ID NOS: 79, 81 and 83.
(13) The DNA according to (11) or (12), wherein the region that encodes the variable region of the heavy chain comprises a nucleotide sequence of SEQ ID NO: 71, and the region that encodes the variable region of the light chain comprises a nucleotide sequence of SEQ ID NO: 69.
(14) The DNA according to (11) or (12), wherein a region that encodes the variable region of the heavy chain comprises a nucleotide sequence of SEQ ID NO: 103, and a region that encodes the variable region of the light chain comprises a nucleotide sequence of SEQ ID NO: 105.
(15) A recombinant vector, comprising the DNA according to any one of (11) to (14).
(16) A transformant, comprising the recombinant vector according to (15).
(17) A hybridoma which produces the monoclonal antibody according to any one of (1) to (10).
(18) A pharmaceutical composition, comprising the monoclonal antibody according to any one of (1) to (10).
(19) The pharmaceutical composition according to (18), which is a composition for cancer treatment.
(20) The pharmaceutical composition according to (19), wherein the composition for cancer treatment is a composition for treatment of one or two or more cancers selected from the group consisting of non-small cell lung cancer, pancreatic cancer and gastric cancer.
(21) The pharmaceutical composition according to any one of (18) to (20), wherein the monoclonal antibody is anchored on a surface of a liposome in which a toxin or an anti-cancer drug is encapsulated.
(22) A diagnostic reagent, which comprises the monoclonal antibody according to any one of (1) to (10).

### BRIEF DESCRPITION OF THE DRAWINGS

Fig. 1 is a diagram (photograph) showing the reactivity of the 020630-18-1 antibody to various tissue slices.
Fig. 2 is a diagram (photograph) showing the binding activity of the 1F8 antibody to the surface of living lung cancer cell and the surface of living normal lung cell, respectively.
Fig. 3 is a diagram showing anti-proliferative effects of the 1F8 antibody on the cultured cancer cell lines HLC-1, PANC-1, and MKN45, respectively.
Fig. 4 is a diagram (photograph) showing the binding activity of γ-1F8 antibody to the surface of fixed cancer cell.

### DETAILED DESCRIPTION OF THE PREFERRED EMBODIMENTS

Hereinafter, the present invention will be described in detail.

### <1> Monoclonal antibody of the present invention

The monoclonal antibody of the present invention is a monoclonal antibody that specifically recognizes a cancer cell-specific antigen.
Specific examples of the monoclonal antibody of the present invention include antibodies in which variable region of the heavy chain comprises amino acid sequences of SEQ ID NOS: 74, 76 and 78 and variable region of the light chain comprises amino acid sequences of SEQ ID NOS: 80, 82 and 84. In the amino acid sequence of SEQ ID NO: 76, the amino acid at position 2 may be Ile or Thr, and the amino acid at position 16 may be Lys or Asn. That is, there are four kinds of sequences of (2Ile, 16Lys), (2Ile, 16Asn), (2Thr, 16Lys) and (2Thr, 16An) as a variable region of the heavy chain.
The above six kinds of sequences are the sequences of the regions called as "hypervariable regions" in variable regions of the heavy and light chain. An antibody consists of heavy chains and light chains, and each of these chains is composed of a constant region and a variable region. A variable region comprises hypervariable regions which determine the specificity of immunoglobulin as an antibody and the binding affinity of the antibody to an epitope. Therefore, regions other than hypervariable regions may be derived from any of other antibodies, so far as hypervariable regions of the present invention comprise each of the above sequences. Here, the term "other antibodies" includes antibodies derived from organisms other than human, but antibodies of human origin are preferable in terms of reducing side effects.
In the present invention, a particularly preferable monoclonal antibody may be one comprising an amino acid sequence of SEQ ID NO: 72 in variable region of the heavy chain and an amino acid sequence of SEQ ID NO: 70 in variable region of the light chain. Here, in the sequence of SEQ ID NO: 72, the amino acid at position 51 may be Ile or Thr, and the amino acid at position 65 may be Lys or Asn.
The antibody of the present invention may have substitution, deletion, or addition of one or several amino acids in the sequence of SEQ ID NO: 72 (heavy chain) and 70 (light chain) as long as the antibody has the specificity that it specifically recognizes a cancer cell-specific antigen. Here, the term "several" means preferably two to five, more preferably two to three, particularly preferably two. Such a substitution, deletion, or addition may be introduced into hypervariable regions, but preferably introduced into regions other than hypervariable regions in variable regions.

In the present invention, the term "monoclonal antibody" refers to any of those including monoclonal antibodies and fragments thereof, F(ab')₂ antibodies, F(ab') antibodies, short-chain antibodies (scFv), diabodies, and minibodies. When the monoclonal antibody contains a constant region, amino acid sequences of its constant regions of the heavy and light chains are preferably one of those described in Nucleic Acids Research vol. 14, p1779, 1986; The Journal of Biological Chemistry vol. 257, p1516, 1982; and Cell vol. 22, p197, 1980. The antibody of the present invention comprising constant and variable regions may be, for example, one comprising an amino acid sequence of SEQ ID NO: 104 (heavy chain) and SEQ ID NO: 106 (light chain).

The monoclonal antibody of the present invention can be obtained by culturing a hybridoma producing the antibody of the present invention in a culture medium, for example, a RPMI1640 medium that contains fetal bovine serum. Alternatively, it can be obtained by preparing a gene (e.g., a gene comprising SEQ ID NO: 103 (heavy chain) or 105 (light chain)), in which a DNA encoding a constant region of heavy chain or light chain is ligated to a DNA encoding each variable region (e.g. DNA comprising SEQ ID NOS: 71 or 69), by means of a PCR method or a chemical synthesis; inserting the obtained gene into a conventionally-used expression vector (e.g., pcDNA3.1 (Invitrogen)) capable of expressing the gene; expressing the gene in a host cell such as a CHO cell (Chinese hamster ovary cell) or *Escherichia coli* to produce the antibody; and purifying the obtained antibody from the culture medium using a Protein A column or the like.

### <2> DNA of the present invention

The DNA of the present invention is a DNA that encodes the monoclonal antibody of the present invention. Examples thereof include a DNA that comprises a region encoding variable region of the heavy chain which comprises amino acid sequences of SEQ ID NOS: 74, 76 and 78; and a region encoding variable region of light chain which comprises amino acid sequences of SEQ ID NOS: 80, 82 and 84. Preferably, such a DNA comprises a region encoding variable region of the heavy chain which comprises nucleotide sequences of SEQ ID NOS: 73, 75 and 77; and a region encoding variable region of the light chain which comprises nucleotide sequences of SEQ ID NOS: 79, 81 and 83. Here, in the sequence of SEQ ID NO: 75, the nucleotide at position 5 may be either "t" or "c", and the nucleotide at position 48 may be either "g" or "t".
The hypervariable regions encoded by those DNA sequences are regions that define the specificity of the antibody, so that sequences encoding the other regions may be those derived from other antibodies. Here, the term "other antibodies" includes antibodies derived from organisms other than human, but, antibodies of human origin are preferable in terms of reducing side effect.
In the present invention, particularly preferable DNA may be one comprising a sequence encoding an amino acid sequence of SEQ ID NO: 72 in variable region of the heavy chain and a sequence encoding an amino acid sequence of SEQ ID NO: 70 in variable region of the light chain. Of those, a particularly preferable DNA may be one comprising a nucleotide sequence of SEQ ID NO: 71 encoding variable region of the heavy chain and a nucleotide sequence of SEQ ID NO: 69 encoding variable region of the light chain. Here, in the sequence of SEQ ID NO: 71, the nucleotide at position 152 may be either "t" or "c", and the nucleotide at position 195 may be either "g" or "t".
Furthermore, the DNA of the present invention may be one which is capable of hybridizing with a DNA comprising nucleotide sequence of SEQ ID NO: 71, and nucleotide sequence of SEQ ID NO: 69 under stringent conditions as long as it encodes a monoclonal antibody that specifically recognizes a cancer cell-specific antigen. Here, stringent conditions include those under which hybridization is performed at a salt concentration corresponding to 60°C, 1 x SSC, 0.1 % SDS, preferably 0.1 X SSC, 0.1% SDS, which corresponds to washing conditions in Southern hybridization.
The DNA of the present invention may be one that encodes all of the constant region and variable region of heavy and light chains. Alternatively, it may be one encoding only the variable regions of the heavy and light chains. When the DNA encodes all of the constant region and variable region, the nucleotide sequences of the constant regions of the heavy and light chains are preferably those described in Nucleic Acids Research vol.14, p1779, 1986, The Journal of Biological Chemistry vol.257, p1516, 1982, and Cell vol.22, p197, 1980. The DNA of the present invention, which encodes the constant region and variable region, encompasses a DNA comprising a nucleotide sequence of SEQ ID NO: 103 (heavy chain) and a nucleotide sequence of SEQ ID NO: 105 (light chain).
The DNA of the present invention can be obtained by the method described below. At first, total RNA is prepared from the cells (e.g., hybridoma cells) of the present invention using a commercially-available RNA extraction kit and then cDNA is synthesized from the total RNA by reverse transcriptase using random primers and the like. Subsequently, using the PCR method in which oligonucleotides each having a sequence conserved in a variable region of heavy chain or light chain of human antibody are used as primers, cDNA encoding such an antibody is amplified. The sequence encoding the constant region can be obtained by amplification of the known sequence by the PCR method. The nucleotide sequence of the DNA can be determined by a conventional method after inserting the DNA into a plasmid for sequence determination. The DNA encoding the monoclonal antibody of the present invention may also be obtained by chemically synthesizing a sequence of variable region or part thereof and linking it to a sequence comprising the constant region.
Furthermore, the present invention provides a recombinant vector comprising the DNA of the present invention, and a transformant containing the recombinant vector. The recombinant vector may be a vector which can be used for gene expression in prokaryotic cells such as *Escherichia coli* (e.g., pBR322, pUC119 or a derivative thereof), preferable is a vector which can be used for gene expression in eukaryotic cells, and more preferable is a vector which can be used for gene expression in cells derived from a mammal. Examples of the vectors which can be used for gene expression in cells derived from a mammal include a plasmid vector such as pcDNA3.1 (manufactured by Invitrogen Co., Ltd.) and a virus vector such as pDON-AI DNA (manufactured by TAKARA BIO INC.). The transformant to be introduced with the recombinant vector of the present invention may be a prokaryotic cell such as *Escherichia coli,* but preferable is a eukaryotic cell, and more preferable is a cell derived from a mammal. Examples of the cells derived from a mammal include a Chinese hamster ovary cell (CHO cell).

### <3> Hybridoma of the present invention

The hybridoma of the present invention is a hybridoma that produces the monoclonal antibody as described above. Examples of the hybridomas of the present invention include hybridoma strains 020630-18-1 and 020630-18-1-1F8-1, which will be explained in the Examples described later. The hybridoma of the present invention can be obtained by the following method. At first, on the basis of the method of A. Imam et. al (Cancer Research vol.45, 263, 1985), tumor-infiltrating lymphocytes are isolated from the tumor tissue removed from a patient diagnosed with gastric cancer and then the cells containing the lymphocytes are fused with mouse myeloma cells using polyethylene glycol to obtain hybridoma cells. Subsequently, enzyme immunoassay is carried out using the supernatant of the obtained hybridoma and then the hybridoma cells that produce antibodies that positively react to various cancer cell lines fixed with paraformaldehyde are selected, followed by cloning the obtained hybridomas by limiting dilution.

### <4> Pharmaceutical composition of the present invention

The pharmaceutical composition of the present invention comprises the monoclonal antibody of the present invention together with a pharmaceutically acceptable carrier. Examples of the pharmaceutically-acceptable carriers include soluble carriers such as known buffers which can be physiologically acceptable (e.g., phosphate buffer) or solid-state carriers such as latex beads.

The pharmaceutical composition of the present invention is suitably used as a therapeutic agent for cancer, particularly for non-small cell lung cancer, pancreatic cancer, and gastric cancer. In addition, the pharmaceutical composition of the present invention may be a composition using the cell-killing effect and anti-proliferative effect of the monoclonal antibody itself, or may be a composition for targeting an anti-cancer drug such as adriamycine to a cancer tissue by binding the anti-cancer drug to the monoclonal antibody of the present invention.

In the present invention, a particularly suitable pharmaceutical composition is one in which the antibody of the present invention is anchored on a liposome containing a toxin, an anti-cancer drug or the like. The liposome used for anchoring the antibody may be composed of a lipid bilayer. Alternatively, the liposome used may be composed of a multiple lipid layers or composed of a single lipid layer. Examples of the constituents of the liposome include phosphatidylcholine, cholesterol and phosphatidyl ethanolamine, and further include phosphatidic acid as a substance for imparting the liposome with electric charge. The ratio of those constituents is, for example, 0.3 to 1 mol, preferably 0.4 to 0.6 mol of cholesterol, 0.01 to 0.2 mol, preferably 0.02 to 0.1 mol of phosphatidylethanolamine, and 0 to 0.4 mol, preferably 0 to 0.15 mol of phosphatidic acid per 1 mol of phosphatidylcholine.

A method of producing the liposome may be any of conventional methods. For instance, it can be produced using a method (Biochimica et Biophysica Acta vol.812, p55, 1985) in which a mixture of the lipids, from which a solvent has been removed, is emulsified by a homogenizer or the like and then subjected to freeze-thawing to obtain a multilamellar liposome, followed by adjustment of pore size of the liposome appropriately by ultrasonication, high-speed homogenization, or pressure filtration through a membrane having uniform-size pores. Preferably, the liposome has a particle size of 30 to 200 nm.

Examples of the pharmaceutical agents to be encapsulated in the liposome include: carcinostatic agents such as adriamycin, daunomycin, mitomycin, cisplatin, vincristine, epirubicin, methotrexate, 5-Fu (5-Fluorouracil) and aclacinomycin; toxins such as ricin A and diphtheria toxin; and antisense RNA. Encapsulation of the agent into liposome may be accomplished by hydration of the lipids with an aqueous solution of the agent. In addition, adriamycin, daunomycin and epirubicin may be encapsulated into the liposome by a remote-loading method using pH gradient (Cancer Res. vol.49 p5922, 1989).

Examples of methods of anchoring the monoclonal antibody on the surface of the liposome include a method in which a purified antibody is coupled with a hydrophobic substance for anchoring the antibody on the liposome, and a method in which an antibody is cross-linked to phosphatidyl ethanolamine using glutaraldehyde. More preferable method is a method in which a liposome containing a lipid into which a maleimide group has been introduced is prepared and an anti-cancer agent or toxin is encapsulated therein, and allowing it to react with a thiolated antibody to thereby anchor the antibody on the surface of the liposome. In addition, a water-soluble polymer derivative that contains a reactive site for an amino group, and a thiol- or intrinsic thiol-group moiety can be preferably used (JP 11-152234A). On the other hand, the surface of the liposome may be modified by allowing the remaining maleimide group to react with a thiolated polyalkylene glycol moiety.

Examples of the methods for introducing a thiol-group into the antibody include a method employing N-succinimidyl-3-(2-pyridyldithio)propionate (SPDP) or compounds such as iminothiolane and mercaptoalkylimidate, which is usually used for thiolation of protein, for introducing a thiol-group to the amino group of the antibody, or a method in which an intrinsic dithiol group of the antibody is reduced to form a thiol group. The method using an intrinsic thiol group is preferable from the view point of maintaining the activity of the antibody. Furthermore, the antibody may be treated with an enzyme such as pepsin to form F(ab')₂ and then reduced with dithiothreitol (DTT) and the like to form F(ab'), which provides one to three thiol groups for binding to the liposome. The binding of the thiolated antibody to the maleimide group-containing liposome may be accomplished by reacting them in a neutral buffer at pH 6.5 to 7.5 for 2 to 16 hours.

The pharmaceutical for cancer treatment of the present invention may be formulated by any of conventional methods such as a dehydration method (JP02-502348A), a method in which a stabilizing agent to obtain a liquid formulation is added (JP64-9331A), and a lyophilization method (JP64-9931A). The pharmaceutical for cancer treatment of the present invention may be administered in intravascularly, intraperitoneally and the like, as local administrations. The dosage thereof can be optimized for the respective drugs encapsulated into the liposome. When the agent is adriamycin, the dosage of adriamycin is 50 mg/kg or less, preferably 10 mg/kg or less, and more preferably 5 mg/kg or less.

### <5> Diagnostic reagent of the present invention

Examples of the diagnostic reagents of the present invention include those taking advantage of the specificity of the antibody of the present invention against cancer cells, particularly a cancer diagnostic reagent comprising the antibody of the present invention, a secondary antibody, a detection substrate, and other components.

### Examples

Hereinafter, the present invention will be described in more detail with reference to examples. However, the present invention is not limited to these examples without departing from the scope of the present invention.

### (1) Preparation of Hybridomas by Cell Fusion between Lymphocytes derived from Regional Lymph Node Cancer from a Cancer Patient and Mouse Myeloma

### (1)-1: Preparation of Lymphocytes

In a plate filled with a culture medium B (culture medium A (RPMI1640 or e-RDF+50 µg/ml gentamicin sulfate) supplemented with 10% fetal calf serum (FCS)), lymphocytes separated from regional lymph node cancer exenterated from a patient with gastric cancer were dispersed on a metal mesh. The cell suspension was centrifuged at 3,000 rpm for 5 minutes and 4.8 × 10⁷ lymphocytes derived from regional lymph node cancer was obtained.

### (1)-2: Cell Fusion

The lymphocytes derived from regional lymph node cancer were fused to mouse myeloma cells (the number thereof being approximately the same as the number of the lymphocytes) using polyethylene glycol 1500 (Roche Diagnostics) according to a standard method (Cancer Research vol. 45, 263, 1985). The fused cells were suspended in a culture medium B so that the cell density becomes 5 × 10⁵ cells/ml. The suspension was dispensed in a 96-well plate at 100 µl/well and cultured at 37°C in CO₂ incubator. On the second day, the culture medium B supplemented with 10 µM hypoxanthine, 0.04 µM aminopterin, and 1.6 µM thymidine (culture medium B supplemented with HAT) was added to each of the wells at 100 µl/well and culture was continued until colonies of hybridomas appeared. As a result, colonies of hybridomas appeared in 23 wells.

### (2) Evaluation of Reactivity of Human Monoclonal Antibody to Cancer Cell Lines

### (2)-1: Cancer Cell Line and its Maintenance

Using the culture supernatant of the obtained hybridomas, reactivity to the fixed gastric cancer cell line MKN45 (Japanese Journal of Cancer and Chemotherapy, vol. 5, p 89, 1978; provided from IBL Co., Ltd.) was tested to select a hybridoma of interest. The cancer cell line was maintained and grown at 37°C under 5% CO₂ in a culture medium D that was prepared by supplementing 5% FCS to culture medium C (D-MEM/F12+50 µg/ml gentamicin sulfate).

### (2)-2: Measurement of Reactivity to Cancer Cell Line

The above-mentioned cancer cell line was cultured in a 96-well plate for 3 to 4 days until it formed a monolayer. After the supernatant was removed, the plate was washed once with 10 mM phosphate buffer (pH 7.4, 0.15 M NaCl) (PBS) and cells were fixed with 2% paraformaldehyde at room temperature for 20 minutes. After washing five times with PBS, the plate was blocked with 150 µl/well of PBS solution containing 5% BSA (bovine serum albumin). Then, the plate was washed five times with PBS and 50 µl of the hybridoma culture supernatant was added thereto to react at 37°C for one and half hours. Next, the plate was washed five times with PBS and then added with 50 µl of a horseradish peroxidase-conjugated goat antibody against a human antibody (CAPPEL, 1,000-fold dilution) to react at 37°C for 1 hour. Subsequently, the plate was washed with PBS containing 0.05% of Tween 20 (PBS-T) and then added with 50 µl/well of phosphate-citrate buffer containing o-phenylenediamine dihydrochloride (5.2%) and H₂O₂ (0.015%). Reaction was conducted at room temperature until color development was observed, followed by the measurement of absorbance at 490 nm with a microphotometer (Nihon Intermed). Cloning of hybridoma was conducted by limiting dilution of the cells obtained from the wells in which reactivity was detected, whereby a hybridoma cell line 020630-18-1 was obtained. Hereinafter, a monoclonal antibody obtained from this cell line is referred to as 020630-18-1 antibody. Further, the cloning of hybridoma was repeated, whereby a hybridoma cell line 020630-18-1-1F8-1 was obtained. Hereinafter, a monoclonal antibody obtained from this cell line is referred to as 1F8 antibody.

### (3) Purification and Labeling of 020630-18-1 and 1F8 Monoclonal Antibodies

### (3)-1: Culture of Hybridomas 020630-18-1 and 020630-18-1-1F8-1 and Purification of 020630-18-1 and 1F8 Monoclonal Antibodies

First, fetal calf serum was allowed to pass through a protein A-glass bead column (PROSEP-A) (bio PROCESSING) to prepare a serum from which substances capable of binding to the PROSEP-A were removed. The culture medium A supplemented with 4-10% of this serum was used to culture the hybridoma 020630-18-1 or 020630-18-1-1F8-1. Next, the culture medium in which the hybridoma 020630-18-1 or hybridoma 020630-18-1-1F8-1 had been cultured was loaded onto the PROSEP-A to thereby adsorb a PROSEP-A-adsorbed polypeptide, which was then eluted to purify the PROSEP-A-adsorbed polypeptide. This PROSEP-A-adsorbed polypeptide was used as a 020630-18-1 antibody or a 1F8 antibody in the subsequent procedures. It was considered that the use of the above-mentioned serum for culture enabled to provide the purified 020630-18-1 antibody or the purified 1 F8 antibody with no contamination of substances capable of binding to the PROSEP-A including antibodies derived from serum. The 020630-18-1 antibody and the 1F8 antibody were confirmed to be pure IgG by sodium dodecyl sulfate-polyacrylamide gel electrophoresis (SDS-PAGE) (data not shown).

### (3)-2: Biotinylation of 020630-18-1 Antibody or 1F8 Antibody

After the 020630-18-1 antibody or the 1F8 antibody purified with the PROSEP-A was biotinylated with a biotinylation reagent (Amersham Pharmacia Biotech) according to the manufacturer's instructions, the labeled antibodies were separated from free biotin by gel filtration method.

### (4) Analysis of Reactivity to Various Tissue Sections

### (4)-1: Preparation of Various Tissue Sections

Various cancer cell lines (lung cancer cell lines: HLC-1 (Cancer vol. 67, No. 4, pp 483-492, 1976; provided from Dr. Suzuki in the Medical Department of the Keio University), A549, PC-3, and PC-9 (all of which from IBL Co., Ltd.); pancreatic cancer cell lines: SUIT2 (provided from Dr. Iguchi in National Hospital Kyushu Cancer Center), HPAC (ATCC No. CRL-2119), PANC-1 (ATCC No. CRL-1469), and PK8 (obtained from the Cell Resource Center for Biomedical Research; the Institute of Development, Aging and Cancer of the Tohoku University); gastric cancer cell lines: MKN45, MKN74, and HSC-3 (all of which from IBL Co., Ltd.); and colon cancer cell lines: HT29 (ATCC No. HTB38), DLD-1 (ATCC No. CCL221), LoVo (ATCC No. CCL229), and CoLo205 (ATCC No. CCL222)) were each grown in the culture medium D at 37°C under 5% CO₂ and transplanted at approximately 1 × 10⁶ to 1 × 10⁷ cells subcutaneously in nude mice (CLEA Japan, Inc.) to form tumors. The tumor tissue sections were each prepared by extracting the formed tumors, fixing the tumors with formalin solution according to a standard method, and embedding the tumors with paraffin.

### (4)-2: Detection of Reactivity to Various Tissue Sections

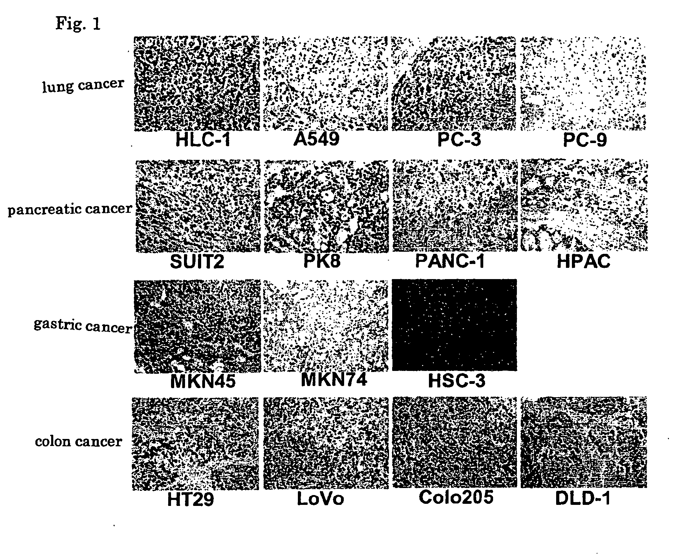

After the prepared various tissue sections were subjected to deparaffinization and blocking procedures according to a standard method, the resulting tissue sections were reacted with the biotinylated 020630-18-1 antibody described in Example (3)-2. DAKO Catalyzed Signal Amplification (CSA) System (DAKO) was used for detection, and reactivity was detected as reddish brown stain by diaminobenzidine. For the immunostained tissue sections, cell nuclei in the tissues were stained in blue with hematoxylin in order to obtain tissue images.
The stained images of various cancer tissue sections of the 020630-18-1 antibody are shown in FIG. 1. It was found that the 020630-18-1 antibody exhibits strong reactivity to the cancer cells in every tumor tissue sections. In particular, nuclei of cancer cells were strongly stained. On the other hand, no reactivity was observed against noncancerous cells in the tissue sections derived from the nude mice.
Further, although not shown in FIG 1, staining was also conducted in the same procedures as described above by using, as a control, a human antibody purified from human serum with the PROSEP-A column in the same way as the 020630-18-1 antibody. The control antibody showed no specific reactivity to each of the tissue sections.

### (5) Binding Activity of 1 F8 Antibody to the Surface of Living Cancer Cells

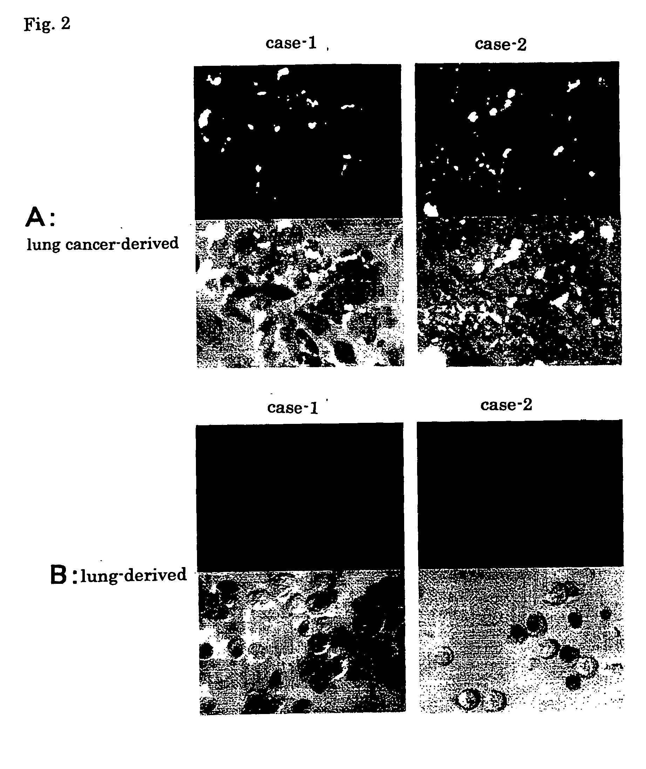

Each nodule of fresh lung cancer tissue and noncancerous lung tissue which was exenterated from a patient with lung cancer by an operation was cut finely with a razor edge, suspended in a culture medium D, passed through a mesh, whereby living cells derived from the tissues were prepared. These living cells were suspended in human serum (containing 0.05% sodium azide), and then the biotinylated 1 F8 antibody described in Example (3)-2 was added to the cell solution so that the concentration of the antibody becomes 50 µg/ml, to adjust the total volume of ∼100 µl (number of cells: 10⁵ to 10⁶). The cell suspension was reacted for 60 minutes under ice-cooling condition. This cell suspension was centrifuged (2,000 rpm, 5 minutes) and the supernatant which was a solution containing the antibody was removed. After that, a 20 nM QdotTM565 streptavidin-labeled solution (manufactured by Quantm Dot Corporation) (containing 0.05% sodium azide) was added and reacted at room temperature for 30 minutes. After removal of the solution, PBS (containing 0.05% sodium azide) was added and cells were observed with a confocal fluorescence microscope (CSU10, manufactured by Yokogawa Electric Corporation). The binding activity of the 1 F8 antibody to the surface of each living cell is shown in FIG. 2. The 1 F8 antibody exhibited binding activity to the surfaces of the living cells derived from the lung cancer tissue (FIG. 2-A), but no binding activity to the living cells derived from the noncancerous lung tissue (FIG. 2-B). The human antibody used as a control exhibited no binding activity to all of the cells (data not shown in FIGS. 2).

### (6) Analysis of the Effects on Cancer Cell Lines

### (6)-1: Cancer Cell Lines and Vascular Endothelial Cells and their Maintenance

As human cancer cell lines, a lung cancer cell line HLC-1, a gastric cancer cell line MKN45, and a pancreatic cancer cell line PANC-1 were used. These cancer cell lines were maintained and grown at 37°C under 5% CO₂ in a culture medium D.

### (6)-2: Analysis of the Anti-Proliferative Effect on Cancer Cell Lines

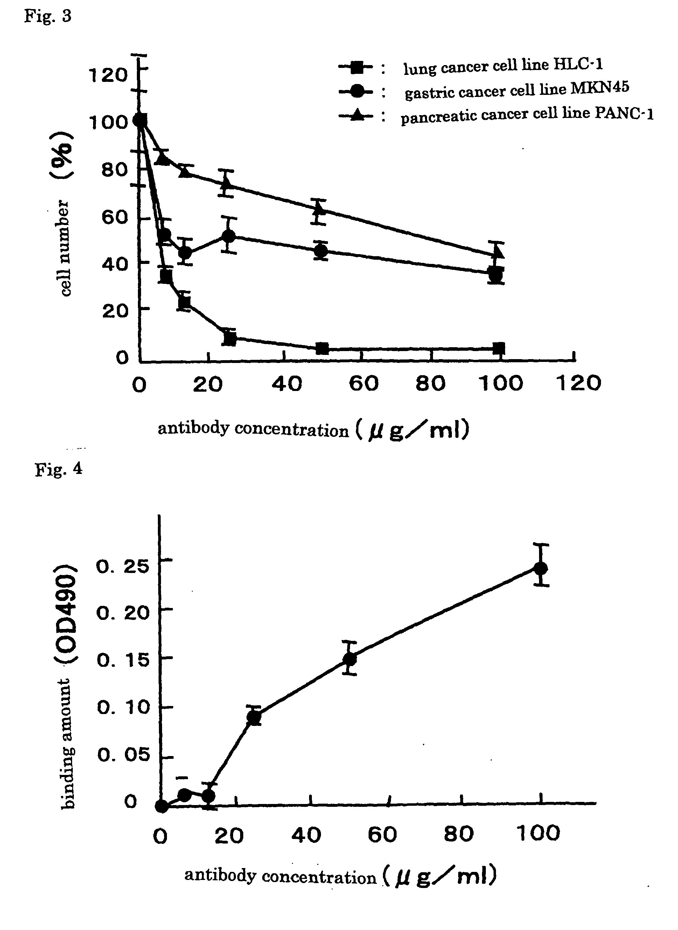

The lung cancer cell line HLC-1, the gastric cancer cell line MKN45, and the pancreatic cancer cell line PANC-1 were each diluted with the culture medium C containing 10% human serum and adjusted so that the number of cells in the well becomes 1.5 × 10³ cells/100 µl/well and the concentration of 1F8 antibody is serially diluted from 100 µg/ml. These cancer cell lines were cultured at 37°C under 5% CO₂, the culture supernatant was changed so as to be the same conditions as described above once every other day, three times in total, and on the 6th day, the number of living cancer cells was compared by MTT assay (J. Immunol. Methods vol.70, p257, 1984).
The result is shown in FIG. 3. Anti-proliferative effects were shown by defining the number of cells under control conditions without the addition of the antibody as 100%. The anti-proliferative effects in a concentration-dependent manner were observed in all cancer cell lines by addition of the 1F8 antibody. In particular, the antibody showed a strong anti-proliferative activity to the lung cancer cell line HLC-1. Moreover, no anti-proliferative effect on the cancer cell lines by the addition of the antibody of the same concentration was observed when the human antibody was used as a control (not shown).

### (7) Acquisition of a Gene Encoding Human Monoclonal Antibody 1F8 and Determination of its Nucleotide Sequence

Total RNA was prepared from the 1F8 antibody-producing hybridoma using an RNeasy Protect Mini kit (QIAGEN). Reverse transcription reaction was performed using ThermoScript RT-PCR System (Invitrogen Corporation) and random hexamers as primers to synthesize cDNA.
Based on the known antibody sequence (J Mol Biol. vol. 222, pp 581-597, 1991), for amplifying variable region of the heavy chain, a mixture of equal amounts of PCR primers (5' end-primers) including VH1 (SEQ ID NO: 1), VH2 (SEQ ID NO: 3), VH3 (SEQ ID NO: 5), VH4 (SEQ ID NO: 7), VH5 (SEQ ID NO: 9), and VH6 (SEQ ID NO: 11), each corresponding to the N-terminal amino acid sequences (SEQ ID NOS: 2, 4, 6, 8, 10, and 12) conserved in the frame 1 of variable region of the heavy chain of human antibodies and a mixture of equal amounts of PCR primers (3' end-primers) including JH1 (SEQ ID NO: 13), JH2 (SEQ ID NO: 15), JH3 (SEQ ID NO: 17), and JH4 (SEQ ID NO: 19), each corresponding to the C-terminal amino acid sequences (SEQ ID NOS: 14, 16, 18, and 20) conserved in the frame 4 of variable region of the heavy chain of human antibodies were used as primers for PCR amplification.
A mixture of equal amounts of PCR primers (5' end-primers) including VK1 (SEQ ID NO: 21), VK2 (SEQ ID NO: 23), VK3 (SEQ ID NO: 25), VK4 (SEQ ID NO: 27), VK5 (SEQ ID NO: 29), and VK6 (SEQ ID NO: 31) each corresponding to the N-terminal amino acid sequences (SEQ ID NOS: 22, 24, 26, 28, 30, and 32) conserved in the frame 1 of variable region of κ chain of human antibodies and a mixture of equal amounts of PCR primers (3' end-primers) including JK1 (SEQ ID NO: 33), JK2 (SEQ ID NO: 35), JK3 (SEQ ID NO: 37), JK4 (SEQ ID NO: 39), and JK5 (SEQ ID NO: 41) each corresponding to the C-terninal amino acid sequences (SEQ ID NOS: 34, 36, 38, 40, and 42) conserved in the frame 4 of variable region of κ chain of human antibodies were used as primers for amplifying variable region of the κ chain.
A mixture of equal amounts of PCR primers (5' end-primers) including VL1 (SEQ ID NO: 43), VL2 (SEQ ID NO: 45), VL3 (SEQ ID NO: 47), VL4 (SEQ ID NO: 49), VL5 (SEQ ID NO: 51), VL6 (SEQ ID NO: 53), and VL7 (SEQ ID NO: 55) each corresponding to the N-terminal amino acid sequences (SEQ ID NOS: 44, 46, 48, 50, 52, 54, and 56) conserved in the frame 1 of variable region of λ chain of human antibodies and a mixture of equal amounts of PCR primers (3' end-primers) including JL1 (SEQ ID NO: 57), JL2 (SEQ ID NO: 59), and JL3 (SEQ ID NO: 61) each corresponding to the C-terminal amino acid sequences (SEQ ID NOS: 58, 60, and 62) conserved in the frame 4 of variable region of λ chain of human antibodies were used as primers for amplifying variable region of the λ chain.
PCR was performed using Perkin Elmer Gene Amp PCR System 2400 and ThermoScript RT-PCR System (Invitrogen Corporation) according to the manufacturer's instructions. As a result, the light chain was amplified by PCR with the primers for the λ chain but not amplified with the primers for the κ chain. Therefore, it was revealed that the light chain of the antibody produced by the hybridoma was a λ chain.
The amplified DNA fragments each encoding variable region of the heavy or light chain were purified using MiniElute PCR Purification kit (QIAGEN). Next, using TOPO TA cloning kit (Invitrogen Corporation), the purified PCR products were ligated to pCR2.1-TOPO, and the transformation of E. *coli* was performed. Appeared colonies were picked up and plasmids were purified using QIAGEN Plasmid mini kit (QIAGEN), followed by digestion with EcoRI at 37°C for 30 minutes and 2% agarose electrophoresis to confirm the insertion of a DNA fragment of interest.
The nucleotide sequences of the inserted DNA fragments were analyzed for several colonies by CEQ 2000 DNA Analysis System (Beckman) using M13 primers. As a result, three sequences including 1F8-H-A (SEQ ID NO: 63), 1F8-H-B (SEQ ID NO: 65), and 1F8-H-C (SEQ ID NO: 67) were identified from the analysis of the heavy chain. A sequence of 1F8-L (SEQ ID NO: 69) was identified from the analysis of the λ chain. By comparing these nucleotide sequences and the corresponding amino acid sequences (heavy chain: SEQ ID NOS: 64, 66, and 68; λ chain: SEQ ID NO: 70), the nucleotide sequences of the variable regions (heavy chain: SEQ ID NO: 71, light chain: SEQ ID NO: 69) and the amino acid sequences (heavy chain: SEQ ID NO: 72, light chain: SEQ ID NO: 70) were determined for each of the heavy and light chains.
A boundary between the hypervariable region (CDR) and the framework was determined with reference to the literature of Kabat et. al. (Sequences of Proteins of Immunological Interest, fifth edition, National Institutes of Health, Bethesda, MD., 1991). As a result, the CDR's of the heavy chain were determined to be HCDR1 (nucleotide sequence: SEQ ID NO: 73, amino acid sequence: SEQ ID NO: 74), HCDR2 (nucleotide sequence: SEQ ID NO: 75, amino acid sequence: SEQ ID NO: 76), and HCDR3 (nucleotide sequence: SEQ ID NO: 77, amino acid sequence: SEQ ID NO: 78). The CDR's of the light chain were determined to be LCDR1 (nucleotide sequence: SEQ ID NO: 79, amino acid sequence: SEQ ID NO: 80), LCDR2 (nucleotide sequence: SEQ ID NO: 81, amino acid sequence: SEQ ID NO: 82), and LCDR3 (nucleotide sequence: SEQ ID NO: 83, amino acid sequence: SEQ ID NO: 84).

### (8) Preparation of γ-1F8. (Recombinant 1F8 Antibody)

### (8-1) Preparation of cDNA and Acquisition of 1F8 Variable Region Fragment

The preparation of cDNA from the total RNA (described in Example (7)) and the PCR were carried out using Perkin Elmer Gene Amp PCR System 2400 and ThermoScript RT-PCR System, High Fidelity (Invitrogen) according to the manufacturer's instructions. Random hexamers supplied in the kit were used as primers for preparing the cDNA of the heavy chain.
For amplification of the variable regions of the heavy chain and the λ chain, primers having a restriction enzyme site for ligation to an expression plasmid were used. For amplification of the variable region of the heavy chain, P1 (SEQ ID NO: 89) containing a AflII site at the 5' terminal and P2 (SEQ ID NO: 90) containing a NheI site at the 3' terminal were used. For amplification of the variable region of the λ chain, P3 (mixture of equal amounts of SEQ ID NO: 91, 92, 93, 94, 95, 96, and 97) containing a BsrGI site at the 5' terminal and P4 (mixture of equal amounts of SEQ ID NO: 98, 99, and 100) containing a AvrII site at the 3' terminal were used.
The DNA fragment of the variable region of each of the amplified heavy chain and the λ chain was purified using QIAquick PCR Purification Kit (QIAGEN). Next, in order to ligate the DNA fragments to the expression vector, the DNA fragments were digested with restriction enzymes. The DNA fragment of the heavy chain was digested with AflII and NheI at 37°C for 2 hours. The DNA fragment of the λ chain was digested with BsrGI and AvrII at 37°C for 2 hours. The digested DNA fragments were subjected to 1.5% agarose electrophoresis to purify a DNA fragment of interest using a QIAquick Gel Extraction Kit (QIAGEN).

### (8-2) Cleavage of Expression Plasmid and Acquisition of Plasmid Fragment

For expressing the human IgG1-type recombinant antibody of the 1F8, pEX-G1-sig-dhfr" containing the sequence of the constant region of the heavy chain and the sequence of the dhfr gene were used as a plasmid for expressing the heavy chain, and pEX-lambda-sig containing the sequence of the constant region of the λ chain was used as a plasmid for expressing the λ chain. The pEX-G1-sig-dhfr" was digested with AflII and SpeI at 37°C for 2 hours. The pEX-lambda-sig was digested with BsrGI and AvrII for 2 hours.
The digested fragments were subjected to 0.8% agarose electrophoresis to purify a fragment of interest using QIAquick Gel Extraction Kit (QIAGEN).

### (8-3) Ligation of 1 F8 Variable Region to the Expression Plasmid

The DNA fragment containing the variable region of the heavy chain of the I F8 obtained by the above-mentioned method was ligated to the cleaved expression plasmid pEX-G1-sig-dhfr" using Ligation Kit Ver. 2.1 (TAKARA) according to the manufacturer's instructions, and the obtained plasmid was used to transform E. *coli* DH5α-T1 (Invitrogen Corporation) according to the manufacturer's instructions. The appeared colonies were picked up and the plasmid was purified using QIAprep Spin Miniprep Kit (QIAGEN). The plasmid containing the nucleotide sequence of the heavy chain was digested with HindIII and NheI at 37°C for 1.5 hours and subjected to 1% agarose electrophoresis, whereby a desired plasmid pEX-1F8-H was obtained. For the obtained plasmid, the nucleotide sequence was confirmed using P5 (SEQ ID NO: 101) at the 5' terminal and P6 (SEQ ID NO: 102) at the 3' terminal. The nucleotide sequence of the structural gene of the heavy chain of the 1F8 recombinant antibody is shown in SEQ ID NO: 103.
On the other hand, the DNA fragment containing the variable region of the λ chain of the 1F8 was ligated to the cleaved expression plasmid pKS-lambda-sig using Ligation Kit Ver. 2.1 (TAKARA) according to the manufacturer's instructions, and the obtained plasmid was used to transform E. *coli* DH5α-T1 (Invitrogen). The appeared colonies were picked up and the plasmid was purified using QIAprep Spin Miniprep Kit (QIAGEN). The plasmid containing the nucleotide sequence of the λ chain was digested with BsrGI and AvrII at 37°C for 2 hours and subjected to 1% agarose electrophoresis, whereby a desired plasmid pEX-1F8-L was obtained. For the obtained plasmid, the nucleotide sequence was confirmed using primer P5 at the 5' terminal. The nucleotide sequence of the structural gene of the λ chain of the 1 F8 recombinant antibody is shown in SEQ ID NO: 105.

### (8-3) Ligation of a 1F8 Heavy Chain to a Plasmid Containing 1F8 λ Chain

For ligating the heavy chain and λ chain of 1F8 to one plasmid, the plasmids obtained above were digested with restriction enzymes. The pEX-1F8-H was digested with NheI at 37°C for 1.5 hours and the pEX-1F8-L was digested with NheI and SpeI at 37°C for 1.5 hours. The resulting fragments were purified using QIAquick PCR Purification Kit (QIAGEN). The fragments were ligated with each other using Ligation Kit Ver. 2.1 (TAKARA) according to the manufacturer's instructions, and the obtained plasmid was used to transform E. *coli* DH5α-T1 (Invitrogen). The appeared colonies were picked up and the plasmid was purified using QIAprep Spin Miniprep Kit (QIAGEN). The obtained plasmid was digested with NheI at 37°C for 1 hour and subjected to 1.5% agarose electrophoresis, whereby a plasmid of a desired size was selected. Further, they were digested with HindIII at 37°C for 1 hour and subjected to 1% agarose electrophoresis to be checked, whereby a desired plasmid pEX-1F8-HL was obtained.

### (8-4) Preparation of γ-1F8-Producing Recombinant

CHO (DG325) cell lines culturable in a serum-free medium were used as cell lines for the preparation of γ-1F8-producing recombinant. The CHO (DG325) were diluted in CHO-S-SFMII (GIBCO) so as to be 1 × 10⁶ cells/ml. Thereafter, 2 µg of plasmid pEX-1F8-HL DNA and 6 µl of FuGENE 6 (Roche) were mixed to transfect the CHO (DG325) according to the manufacturer's instructions. At 5.5 hours after transfection, EX-CELL325-PF (Nichirei) was added. After two-day culture, 400 µg/ml of G418 (Promega) and 0 or 25 nM of methotrexate (Sigma) were added to the medium as selection reagents and culture was conducted, followed by cloning, whereby γ-1F8-producing cell line was obtained.

### (8-5) Screening of Antibody-Producing Cell Line using ELISA Assay

The screening of the antibody-producing cell was conducted by sandwich ELISA assay. First, a goat anti-human immunoglobulin antibody (CAPPEL) was diluted to 50 µg/ml with PBS and put into a 96-well plate (FALCON) at 50 µl/well to be incubated at 37°C for 2 hours. After washed five times with PBS, the plate was added with 150 µl/well of PBS solution containing 5% bovine serum albumin (BSA) and blocking was performed. The plate was washed with PBS five times and then added with 50 µl of culture supernatant of the recombinant to react at 37°C for 2 hours. Next, the plate was washed with PBS five times and then added with 50 µl of horseradish peroxidase (HRP)-conjugated goat antibody against a human antibody (1,000-fold dilution, CAPPEL) to react at 37°C for 1 hour. Next, the plate was washed with PBS containing 0.05% of Tween 20 (PBS-T) and then added with 50 µl/well of phosphate-citrate buffer containing o-phenylenediamine dihydrochloride (5.2%) and H₂O₂ (0.015%). Reaction was conducted at room temperature until color development was observed, followed by stopping the reaction by adding 50 µl/well of 1N-H₂SO₄. The measurement of absorbance at 490 nm with a microphotometer (Nihon Intermed) was performed, and a cell line having the largest amount of production was selected.

### (8-6) Purification of γ-1 F8

The γ-1F8-producing cell line obtained above was cultured in a culture medium A containing 4% serum treated with PROSEP-A and added with G418 (Promega), whereby a culture medium containing the γ-1F8 was obtained. Next, the culture medium was loaded onto the PROSEP-A for adsorbing the γ-1F8 on it. The adsorbed γ-1F8 was then eluted whereby the γ-1F8 was purified. It was confirmed that the γ-1F8 was a pure IgG by SDS-PAGE (not shown).

### (8-7) Confirmation of Binding Activity of γ-1F8 to Cancer Cell Line

The binding activity of the purified γ-1F8 described in the item (8-6) to the HLC-1 cell which was fixed by the same method as described in the item (2-2) was confirmed. The antibodies having respective concentrations were reacted with the fixed cancer cell line at 37°C for 1 hour. The plate was washed with PBS, added with HRP-conjugated goat antibody against a human antibody (1,000-fold dilution, CAPPEL) to react at 37°C for 1 hour. The reactivity of γ-1F8 was detected, and as a result, it was confirmed that γ-1F8 exhibited binding activity to HLC-1 cell in a concentration-dependent manner (FIG. 4).

### INDUSTRIAL APPLICABILITY

By using a monoclonal antibody obtained in the present invention, an anti-cancer drug that selectively attacks cancer tissues, particularly non-small cell lung cancer, pancreatic cancer or gastric cancer can be provided. Moreover, when the human monoclonal antibody of the present invention is used, an anti-cancer drug capable of continuous administration with reduced side effects can be provided.

## Claims

1. A monoclonal antibody, wherein variable region of heavy chain of said monoclonal antibody comprises amino acid sequences of SEQ ID NOS: 74, 76 and 78.

2. The monoclonal antibody according to claim 1, wherein the variable region of the heavy chain comprises an amino acid sequence of SEQ ID NO: 72.

3. The monoclonal antibody according to claim 1, wherein the variable region of the heavy chain comprises an amino acid sequence of SEQ ID NO: 104.

4. A monoclonal antibody, wherein variable region of the light chain of said monoclonal antibody comprises amino acid sequences of SEQ ID NOS: 80, 82 and 84.

5. The monoclonal antibody according to claim 4, wherein the variable region of the light chain comprises an amino acid sequence of SEQ ID NO: 70.

6. The monoclonal antibody according to claim 4, wherein the variable region of the light chain comprises an amino acid sequence of SEQ ID NO: 106.

7. A monoclonal antibody, wherein variable region of the heavy chain of said monoclonal antibody comprises amino acid sequences of SEQ ID NOS: 74, 76 and 78, and variable region of the light chain of said monoclonal antibody comprises amino acid sequences of SEQ ID NOS: 80, 82 and 84.

8. The monoclonal antibody according to claim 7, wherein the variable region of the heavy chain contains an amino acid sequence of SEQ ID NO: 72, and the variable region of the light chain comprises an amino acid sequence of SEQ ID NO: 70.

9. The monoclonal antibody according to claim 7, wherein the variable region of the heavy chain comprises an amino acid sequence of SEQ ID NO: 104, and the variable region of the light chain comprises an amino acid sequence of SEQ ID NO: 106.

10. The monoclonal antibody according to any one of claims 1 to 9, wherein said monoclonal antibody is a human antibody.

11. A DNA which encodes the monoclonal antibody according to any one of claims 1 to 10.

12. The DNA according to claim 11, wherein a region that encodes variable region of the heavy chain comprises nucleotide sequences of SEQ ID NOS: 73, 75 and 77, and a region that encodes variable region of the light chain comprises nucleotide sequences of SEQ ID NOS: 79, 81 and 83.

13. The DNA according to claim 11 or 12, wherein the region that encodes the variable region of the heavy chain comprises a nucleotide sequence of SEQ ID NO: 71, and the region that encodes the variable region of the light chain comprises a nucleotide sequence of SEQ ID NO: 69.

14. The DNA according to claim 11 or 12, wherein a region that encodes the variable region of the heavy chain comprises a nucleotide sequence of SEQ ID NO: 103, and a region that encodes the variable region of the light chain comprises a nucleotide sequence of SEQ ID NO: 105.

15. A recombinant vector, comprising the DNA according to any one of claims 11 to 14.

16. A transformant, comprising the recombinant vector according to claim 15.

17. A hybridoma which produces the monoclonal antibody according to any one of claims 1 to 10.

18. A pharmaceutical composition, comprising the monoclonal antibody according to any one of claims 1 to 10.

19. The pharmaceutical composition according to claim 18, which is a composition for cancer treatment.

20. The pharmaceutical composition according to claim 19, wherein the composition for cancer treatment is a composition for treatment of one or two or more cancers selected from the group consisting of non-small cell lung cancer, pancreatic cancer and gastric cancer.

21. The pharmaceutical composition according to any one of claims 18 to 20, wherein the monoclonal antibody is anchored on a surface of a liposome in which a toxin or an anti-cancer drug is encapsulated.

22. A diagnostic reagent, which comprises the monoclonal antibody according to any one of claims 1 to 10.
